# EUROPEAN PATENT APPLICATION

(11) **EP 2 223 701 A1**
(43) Date of publication of application: **01.09.2010**
(21) Application number: 08846883.0
(22) Date of filing: 07.11.2008
(51) Int. Cl.: A61K 39/395, A61K 38/21, C07K 16/28

(54) **PHARMACEUTICAL COMPOSITION FOR CANCER TREATMENT**

(30) Priority: 07.11.2007 ES 200702961
(71) Applicant: Proyecto de Biomedicina Cima, S.L., 31180 Cizur Mayor - Navarra (ES); Institut National de la Santé et de la Recherche Médicale, 75654 Paris Cedex 13 (FR)
(72) Inventor: MELERO BERMEJO, Ignacio Javier, E-31008 Pamplona - Navarra (ES); DUBROT ARMENDÁRIZ, Juan, E-31008 Pamplona - Navarra (ES); HERVÁS STUBBS, Sandra, E-31008 Pamplona - Navarra (ES); LE BON, Agnes Laurence, F-75014 Paris (FR)
(74) Representative: ABG Patentes, S.L.
(86) International application number: PCT/ES2008/000693
(87) International publication number: WO 2009/060112

(57) **Abstract**

The invention relates to therapeutic compositions for the treatment of cancer and, more specifically, to compositions comprising a 4-1BB receptor agonist ligand and a type I interferon, whose simultaneous or sequential administration results in a synergic antitumoral effect compared to the administration of either of the components individually. The present invention also relates to the therapeutic uses of the combinations of the said invention for the treatment of cancer. Finally, the invention relates to the polynucleotide that codes for both compounds respectively, vectors and cells that comprise said polynucleotide as well as their use for the treatment of cancer.

SEQUENCE LISTING

<110>  Proyecto de Biomedicina CIMA, S.L.
       Institut National de la Santé et de la Reserche Médicale

<120>  PHARMACEUTICAL COMPOSITION FOR THE TREATMENT OF CANCER

<130>  P3297PC00

<150>  P200702961.8
<151>  2007-11-07

<160>  7

<170>  PatentIn version 3.5

<210>  1
<211>  1619
<212>  DNA
<213>  H.sapiens

<400>  1
gtcatggaat acgcctctga cgcttcactg gaccccgaag ccccgtggcc tcccgcgccc      60

cgcgctcgcg cctgccgcgt actgccttgg gccctggtcg cggggctgct gctgctgctg     120

ctgctcgctg ccgcctgcgc cgtcttcctc gcctgcccct gggccgtgtc cggggctcgc     180

gcctcgcccg gctccgcggc cagcccgaga ctccgcgagg gtcccgagct ttcgcccgac     240

gatcccgccg gcctcttgga cctgcggcag ggcatgtttg cgcagctggt ggcccaaaat     300

gttctgctga tcgatgggcc cctgagctgg tacagtgacc caggcctggc aggcgtgtcc     360

ctgacggggg gcctgagcta caaagaggac acgaaggagc tggtggtggc caaggctgga     420

gtctactatg tcttctttca actagagctg cggcgcgtgg tggccggcga gggctcaggc     480

tccgtttcac ttgcgctgca cctgcagcca ctgcgctctg ctgctggggc cgccgccctg     540

gctttgaccg tggacctgcc acccgcctcc tccgaggctc ggaactcggc cttcggtttc     600

cagggccgct tgctgcacct gagtgccggc cagcgcctgg gcgtccatct tcacactgag     660

gccagggcac gccatgcctg gcagcttacc cagggcgcca cagtcttggg actcttccgg     720

gtgacccccg aaatcccagc cggactccct tcaccgaggt cggaataacg cccagcctgg     780

gtgcagccca cctggacaga gtccgaatcc tactccatcc ttcatggaga cccctggtgc     840

tgggtccctg ctgctttctc tacctcaagg ggcttggcag gggtccctgc tgctgacctc      900

cccttgagga ccctcctcac ccactccttc cccaagttgg accttgatat ttattctgag     960

cctgagctca gataatatat tatatatatt atatatatat atatatttct atttaaagag    1020

gatcctgagt ttgtgaatgg acttttttag aggagttgtt ttgggggggg ggtcttcgac     1080

attgccgagg ctggtcttga actcctggac ttagacgatc ctcctgcctc agcctcccaa 1140

gcaactggga ttcatccttt ctattaattc attgtactta tttgcctatt tgtgtgtatt 1200

gagcatctgt aatgtgccag cattgtgccc aggctagggg gctatagaaa catctagaaa 1260

tagactgaaa gaaaatctga gttatggtaa tacgtgagga atttaaagac tcatccccag 1320

cctccacctc ctgtgtgata cttgggggct agcttttttc tttctttctt ttttttgaga 1380

tggtcttgtt ctgtcaacca ggctagaatg cagcggtgca atcatgagtc aatgcagcct 1440

ccagcctcga cctcccgagg ctcaggtgat cctcccatct cagcctctcg agtagctggg 1500

accacagttg tgtgccacca cacttggcta actttttaat ttttttgcgg agacggtatt 1560

gctatgttgc caaggttgtt tacatgccag tacaatttat aataaacact catttttcc 1619

<210> 2
<211> 1239
<212> DNA
<213> Mus musculus

<400> 2
agcctataaa gcacgggcac tggcgggaga cgtgcactga ccgaccgtgg taatggacca 60

gcacacactt gatgtggagg ataccgcgga tgccagacat ccagcaggta cttcgtgccc 120

ctcggatgcg gcgctcctca gagataccgg gctcctcgcg gacgctgcgc tcctctcaga 180

tactgtgcgc cccacaaatg ccgcgctccc cacggatgct gcctaccctg cggttaatgt 240

tcgggatcgc gaggccgcgt ggccgcctgc actgaacttc tgttcccgcc acccaaagct 300

ctatggccta gtcgctttgg ttttgctgct tctgatcgcc gcctgtgttc ctatcttcac 360

ccgcaccgag cctcggccag cgctcacaat caccacctcg cccaacctgg gtacccgaga 420

gaataatgca gaccaggtca cccctgtttc ccacattggc tgccccaaca ctacacaaca 480

gggctctcct gtgttcgcca agctactggc taaaaaccaa gcatcgttgt gcaatacaac 540

tctgaactgg cacagccaag atggagctgg gagctcatac ctatctcaag gtctgaggta 600

cgaagaagac aaaaaggagt tggtggtaga cagtcccggg ctctactacg tatttttgga 660

actgaagctc agtccaacat tcacaaacac aggccacaag gtgcagggct gggtctctct 720

tgttttgcaa gcaaagcctc aggtagatga ctttgacaac ttggccctga cagtggaact 780

gttcccttgc tccatggaga acaagttagt ggaccgttcc tggagtcaac tgttgctcct 840

gaaggctggc caccgcctca gtgtgggtct gagggcttat ctgcatggag cccaggatgc 900

atacagagac tgggagctgt cttatcccaa caccaccagc tttggactct ttcttgtgaa 960

acccgacaac ccatgggaat gagaactatc cttcttgtga ctcctagttg ctaagtcctc 1020

aagctgctat gttttatggg gtctgagcag gggtcccttc catgactttc tcttgtcttt 1080

aactggactt ggtatttatt ctgagcatag ctcagacaag actttatata attcactaga 1140

tagcattagt aaactgctgg gcagctgcta gataaaaaaa aatttctaaa tcaaagttta 1200

tatttatatt aatatataaa aataaatgtg tttgtaaat 1239

<210> 3
<211> 254
<212> PRT
<213> H.sapiens

<400> 3
   Met Glu Tyr Ala Ser Asp Ala Ser Leu Asp Pro Glu Ala Pro Trp Pro
   1 5 10 15
   Pro Ala Pro Arg Ala Arg Ala Cys Arg Val Leu Pro Trp Ala Leu Val
   20 25 30
   Ala Gly Leu Leu Leu Leu Leu Leu Leu Ala Ala Ala Cys Ala Val Phe
   35 40 45
   Leu Ala Cys Pro Trp Ala Val Ser Gly Ala Arg Ala Ser Pro Gly Ser
   50 55 60
   Ala Ala Ser Pro Arg Leu Arg Glu Gly Pro Glu Leu Ser Pro Asp Asp
   65 70 75 80
   Pro Ala Gly Leu Leu Asp Leu Arg Gln Gly Met Phe Ala Gln Leu Val
   85 90 95
   Ala Gln Asn Val Leu Leu Ile Asp Gly Pro Leu Ser Trp Tyr Ser Asp
   100 105 110
   Pro Gly Leu Ala Gly Val Ser Leu Thr Gly Gly Leu Ser Tyr Lys Glu
   115 120 125
   Asp Thr Lys Glu Leu Val Val Ala Lys Ala Gly Val Tyr Tyr Val Phe
   130 135 140
   Phe Gln Leu Glu Leu Arg Arg Val Val Ala Gly Glu Gly Ser Gly Ser
   145 150 155 160
   Val Ser Leu Ala Leu His Leu Gln Pro Leu Arg Ser Ala Ala Gly Ala
                   165                 170                 175
   Ala Ala Leu Ala Leu Thr Val Asp Leu Pro Pro Ala Ser Ser Glu Ala
               180                 185                 190
   Arg Asn Ser Ala Phe Gly Phe Gln Gly Arg Leu Leu His Leu Ser Ala
           195                 200                 205
   Gly Gln Arg Leu Gly Val His Leu His Thr Glu Ala Arg Ala Arg His
       210                 215                 220
   Ala Trp Gln Leu Thr Gln Gly Ala Thr Val Leu Gly Leu Phe Arg Val
   225                 230                 235                 240
   Thr Pro Glu Ile Pro Ala Gly Leu Pro Ser Pro Arg Ser Glu
                   245                 250

<210>  4
<211>  309
<212>  PRT
<213>  Mus musculus

<400>  4

Met Asp Gln His Thr Leu Asp Val Glu Asp Thr Ala Asp Ala Arg His
1               5                   10                  15

Pro Ala Gly Thr Ser Cys Pro Ser Asp Ala Ala Leu Leu Arg Asp Thr
            20                  25                  30

Gly Leu Leu Ala Asp Ala Ala Leu Leu Ser Asp Thr Val Arg Pro Thr
        35                  40                  45

Asn Ala Ala Leu Pro Thr Asp Ala Ala Tyr Pro Ala Val Asn Val Arg
    50                  55                  60

Asp Arg Glu Ala Ala Trp Pro Pro Ala Leu Asn Phe Cys Ser Arg His
65                  70                  75                  80

Pro Lys Leu Tyr Gly Leu Val Ala Leu Val Leu Leu Leu Leu Ile Ala
                85                  90                  95

Ala Cys Val Pro Ile Phe Thr Arg Thr Glu Pro Arg Pro Ala Leu Thr
            100                 105                 110

Ile Thr Thr Ser Pro Asn Leu Gly Thr Arg Glu Asn Asn Ala Asp Gln
        115                 120                 125

Val Thr Pro Val Ser His Ile Gly Cys Pro Asn Thr Thr Gln Gln Gly
    130                 135                 140

Ser Pro Val Phe Ala Lys Leu Leu Ala Lys Asn Gln Ala Ser Leu Cys
145                 150                 155                 160

Asn Thr Thr Leu Asn Trp His Ser Gln Asp Gly Ala Gly Ser Ser Tyr
                165                 170                 175

Leu Ser Gln Gly Leu Arg Tyr Glu Glu Asp Lys Lys Glu Leu Val Val
            180                 185                 190

Asp Ser Pro Gly Leu Tyr Tyr Val Phe Leu Glu Leu Lys Leu Ser Pro
        195                 200                 205

Thr Phe Thr Asn Thr Gly His Lys Val Gln Gly Trp Val Ser Leu Val
    210                 215                 220

Leu Gln Ala Lys Pro Gln Val Asp Asp Phe Asp Asn Leu Ala Leu Thr
225                 230                 235                 240

Val Glu Leu Phe Pro Cys Ser Met Glu Asn Lys Leu Val Asp Arg Ser
                245                 250                 255

Trp Ser Gln Leu Leu Leu Leu Lys Ala Gly His Arg Leu Ser Val Gly
            260                 265                 270

Leu Arg Ala Tyr Leu His Gly Ala Gln Asp Ala Tyr Arg Asp Trp Glu
        275                 280                 285

Leu Ser Tyr Pro Asn Thr Thr Ser Phe Gly Leu Phe Leu Val Lys Pro
    290                 295                 300

Asp Asn Pro Trp Glu
305

<210>  5
<211>  204
<212>  PRT
<213>  H. sapiens

<400>  5

Cys Pro Trp Ala Val Ser Gly Ala Arg Ala Ser Pro Gly Ser Ala Ala
1               5                   10                  15

Ser Pro Arg Leu Arg Glu Gly Pro Glu Leu Ser Pro Asp Asp Pro Ala
            20                  25                  30

Gly Leu Leu Asp Leu Arg Gln Gly Met Phe Ala Gln Leu Val Ala Gln
        35                  40                  45

Asn Val Leu Leu Ile Asp Gly Pro Leu Ser Trp Tyr Ser Asp Pro Gly
    50                  55                  60

Leu Ala Gly Val Ser Leu Thr Gly Gly Leu Ser Tyr Lys Glu Asp Thr
65                  70                  75                  80

Lys Glu Leu Val Val Ala Lys Ala Gly Val Tyr Tyr Val Phe Phe Gln
                85                  90                  95

Leu Glu Leu Arg Arg Val Val Ala Gly Glu Gly Ser Gly Ser Val Ser
            100                 105                 110

Leu Ala Leu His Leu Gln Pro Leu Arg Ser Ala Ala Gly Ala Ala Ala
        115                 120                 125

Leu Ala Leu Thr Val Asp Leu Pro Pro Ala Ser Ser Glu Ala Arg Asn
    130                 135                 140

Ser Ala Phe Gly Phe Gln Gly Arg Leu Leu His Leu Ser Ala Gly Gln
145                 150                 155                 160

Arg Leu Gly Val His Leu His Thr Glu Ala Arg Ala Arg His Ala Trp
                165                 170                 175

Gln Leu Thr Gln Gly Ala Thr Val Leu Gly Leu Phe Arg Val Thr Pro
            180                 185                 190

Glu Ile Pro Ala Gly Leu Pro Ser Pro Arg Ser Glu
        195                 200

<210>  6
<211>  206
<212>  PRT
<213>  Mus musculus

<400>  6

Arg Thr Glu Pro Arg Pro Ala Leu Thr Ile Thr Thr Ser Pro Asn Leu
1               5                   10                  15

Gly Thr Arg Glu Asn Asn Ala Asp Gln Val Thr Pro Val Ser His Ile
            20                  25                  30

Gly Cys Pro Asn Thr Thr Gln Gln Gly Ser Pro Val Phe Ala Lys Leu
        35                  40                  45

Leu Ala Lys Asn Gln Ala Ser Leu Cys Asn Thr Thr Leu Asn Trp His
    50                  55                  60

Ser Gln Asp Gly Ala Gly Ser Ser Tyr Leu Ser Gln Gly Leu Arg Tyr
65                  70                  75                  80

Glu Glu Asp Lys Lys Glu Leu Val Val Asp Ser Pro Gly Leu Tyr Tyr
                85                  90                  95

Val Phe Leu Glu Leu Lys Leu Ser Pro Thr Phe Thr Asn Thr Gly His
            100                 105                 110

Lys Val Gln Gly Trp Val Ser Leu Val Leu Gln Ala Lys Pro Gln Val
        115                 120                 125

Asp Asp Phe Asp Asn Leu Ala Leu Thr Val Glu Leu Phe Pro Cys Ser
    130                 135                 140

Met Glu Asn Lys Leu Val Asp Arg Ser Trp Ser Gln Leu Leu Leu Leu
145                 150                 155                 160

Lys Ala Gly His Arg Leu Ser Val Gly Leu Arg Ala Tyr Leu His Gly
                165                 170                 175

Ala Gln Asp Ala Tyr Arg Asp Trp Glu Leu Ser Tyr Pro Asn Thr Thr
            180                 185                 190

Ser Phe Gly Leu Phe Leu Val Lys Pro Asp Asn Pro Trp Glu
        195                 200                 205

<210>  7
<211>  8
<212>  PRT
<213>  Artificial

<220>
<223> Tumor antigen

<400>  7

Lys Ser Pro Trp Phe Thr Thr Leu
1               5

## Description

### TECHNICAL FIELD OF THE INVENTION

The invention relates to therapeutic compositions for the treatment of cancer and, more specifically, to compositions comprising a 4-1BB receptor agonist ligand and a type I interferon.

### BACKGROUND

The usual medical treatments for cancer, such as chemotherapy, surgery, radiotherapy and cell therapy, currently have clear limitations with respect to the efficacy and toxicity of said treatments. Up until now, these strategies have given rise to different degrees of success depending on the type of cancer, general health of the patient, stage of the disease at the time of diagnosis, etc.

It is known in the state of the art that the immune system has a critical role in the pathogenesis of a wide variety of cancers. It is believed that the progress of cancer is due to a failure in the immune response, which allows the growth of the cancer. A possible strategy in the treatment of cancer consists of stimulating the immune system so it specifically attacks tumor cells.

4-1BB (CD137) is a costimulus receptor that is mainly expressed in the membrane of T-cells or NK (Natural Killer) cells after their activation, respectively, by antigens or cytokines, although it is also found in the surface of other myeloid strain leukocytes. The costimulation signals through this receptor that are induced with the natural 4-1BB-Ligand (4-1BB-L) or with agonist antibodies can be:
(i) enhancing the proliferation and survival of culture stimulated lymphocytes;
(ii) stimulating the expansion of antigen-activated T-cells, particularly CD8+ T-cells;
(iii)favoring the survival (apoptosis inhibition) of said T-cells;
(iv) inducing cytotoxic T (CTL) responses; and
(v) activating the function of Natural Killer (NK) lymphocytes.

The systemic administration of agonist anti-4-1BB antibodies enhances the response of cytotoxic T-cells against tumor antigens, which determines the eradication in mice of transplantable tumors with a certain degree of baseline immunogenicity (Melero et al., Nat Med. 1997. 3(6):682-5), and enhances the activity of vaccination with tumor antigens or the adoptive transfer of T cells with anti-tumor reactivity (Wilcox, R.A. et al., J Clin Invest. 2002. 109(5):651-9). Clinical trials in which a humanized anti-4-1BB monoclonal antibody is being administered to patients who are carriers of different neoplasias are being conducted as a continuation of these discoveries and inventions (clinicaltrials.gov: NTC00309023 and NTC00351325). Patent applications W0200544996 and W02006088447 describe the use of 4-1BB receptor agonists in the treatment of cancer.

Preclinical studies suggest that the treatment with anti-4-1BB antibodies has a safe toxicological profile, although some adverse effects in the form of hepatic lymphocyte infiltration and myelosuppression occur at repeated high doses. There are transplantable mice tumors resistant to the treatment with these anti-4-1BB antibodies used as monotherapy. Therefore, the existence of an immunosuppression or tolerance condition making the immune response against tumor antigens difficult is possible in patients with advanced cancer and high tumor burden.

A number of cytokines have proved to play an essential role in immune response regulation. Type I interferons (IFNs) are a family of polypeptides with cytokine activity which were originally discovered by virtue of their inhibitory activity on the viral infection of cells lines *in vitro* (Pestka, S., Krause, C.D. and Walter, M.R. 2004. Immunol Rev. Vol. 202:8-32). Depending on the homology of their sequences, type I interferons are classified into interferon-α (IFN-α), interferon-β (IFN-β) and interferon-ω (IFN-ω). IFN-α and IFN-β share a single dimeric receptor which is expressed in the surface of most nucleated cells. Both the human and mouse genome contain a single gene encoding IFN-β, whereas they contain 12 or 13 functional genes encoding IFN-α. The function of these cytokines is very important in the immune response against multiple types of viral infections, because they initiate mechanisms promoting the apoptosis-induced death of the infected cells and viral replication inhibition while at the same time it favors antigen presentation. It has recently been experimentally documented that it also carries out its functions by directly activating the activities of NK, B and T-cells, as well as of dendritic cells in the immune response (Le Bon A. et al., 2003. Nat. Immunol. Vol.: 4(10):1009-15; Le Bon A. et al., 2006. J. Immunol. Vol.: 176(8):4682-4689; Le Bon A. et al., 2006. J Immunol. Vol.: 176(4):2074-8).

As a drug, IFN-α is prescribed in the treatment of chronic viral hepatitis and is used in the treatment of several malignant diseases, such as melanoma and chronic myeloid leukemia, for example. Murata, M. et al., 2006. Cytokine, Vol. 33: 121-128, describe the anti-tumor effects of interferons α and β on hepatocellular carcinoma cell lines, such as antiproliferative effect, change of cell cycle and apoptosis. The anti-tumor effect of IFN-α is mediated by direct pro-apoptotic effects on tumor cells, anti-angiogenic effects on vascular tumor cells and enhancing effects on the anti-tumor immune response. However, clinical trials show that the efficacy of IFN-α in the treatment of tumors is very limited, such that the clinical benefit compared to the adverse effects is not very favorable, and therefore its use in oncology has currently been very limited.

There are a number of references describing the treatment of cancer by means of the combined administration of different compounds. Martinet, O. et al. (Journal of the National Cancer Institute, 2000, 92: 931-936) have described hepatic metastasis remission in a mouse model by means of gene therapy, thanks to the administration of an adenovirus carrying the genes encoding interleukin 12 (IL-12) and the 4-1BB ligand. Chen, S. et al. (Molecular Therapy, 2000, 2:39-46) have described an effective anti-tumor CD8+ T-cell response thanks to the combination of gene therapy with IL-12 and the systemic release of an agonist monoclonal antibody against 4-1BB. After the combined treatment of IL-12 and anti-4-1BB, the effective dose of IL-12 could be reduced up to 18 times and a greater efficacy was reached than when the maximum dose of IL-12 is administered alone. Patent application W02004/093831 describes the combined administration of a cytokine-expressing cellular vaccine and at least one therapeutic agent against cancer, wherein the combined administration of both compounds involves an increase in therapeutic efficacy with respect to the efficacy reached when the compounds are administered alone.

For these reasons, the strategies including the combination of the handling of the immune response to cancer with usual medical treatments may be the means of improving the efficacy of the treatment and reducing the toxicity thereof.

### SUMMARY OF THE INVENTION

In one aspect the invention relates to a composition comprising at least one 4-1BB receptor agonist ligand or a functionally equivalent variant thereof and at least one type I interferon or a functionally equivalent variant thereof.

Due to the therapeutic applications of the composition according to the present invention, in another aspect, the invention relates to a composition comprising at least one 4-1BB receptor agonist ligand or a functionally equivalent variant thereof and at least one type I interferon or a functionally equivalent variant thereof for its use in medicine.

In another aspect, the invention relates to the use of a composition according to the invention for preparing a medicament for the treatment or the prevention of cancer.

In another aspect, the invention relates to a pharmaceutical preparation comprising a composition according to the present invention and a pharmaceutically acceptable carrier.

In another aspect, the invention relates to a kit comprising, in one or several containers,
(i) a pharmaceutically acceptable formulation of at least one 4-1BB receptor agonist ligand or a functionally equivalent variant thereof,
(ii) a pharmaceutically acceptable formulation of at least one type I interferon or a functionally equivalent variant thereof, and optionally,
(iii) a pharmaceutically acceptable formulation of at least one chemotherapeutic compound.

The uses of the kit described in the present invention form additional inventive aspects. Therefore, in another aspect, the invention relates to a kit according to the invention for its use in medicine or its use in the preparation of a medicament for the treatment or the prevention of cancer.

In another aspect, the invention relates to the use of a type I interferon or a functionally equivalent variant thereof for promoting the anti-tumor effect of a 4-1BB receptor agonist ligand.

In another aspect, the invention relates to a polynucleotide comprising
(i) a nucleotide sequence encoding a 4-1BB receptor agonist ligand or a functionally equivalent variant thereof, and
(ii) a nucleotide sequence encoding a type I interferon or a functionally equivalent variant thereof,
   wherein both sequences are preceded by expression regulating sequences.

In another aspect, the invention relates to a vector comprising a polynucleotide described in the present invention as well as to a cell comprising a vector of the invention.

The uses of the polynucleotide, vector and cell of the invention form another aspect of the invention. Therefore, in another aspect, the invention relates to a polynucleotide of the invention, a vector of the invention and a cell of the invention for their use in medicine, to the use of the polynucleotide, vector and cell for preparing a medicament for the treatment of cancer as well as to pharmaceutical preparations comprising the polynucleotide of the invention, the vector of the invention and the cell of the invention together with a pharmaceutically acceptable carrier.

In another aspect, the invention relates to a kit comprising a polynucleotide of the invention, a vector of the invention or a cell according to the invention.

In another aspect, the invention relates to a kit comprising a polynucleotide of the invention, a vector of the invention or a cell according to the invention for their use in medicine as well as to the use of said kit for preparing a medicament in the treatment or prevention of cancer.

Finally, the invention relates to the use of a polynucleotide encoding a type I interferon, or a functionally equivalent variant thereof, for promoting the anti-tumor effect of a 4-1BB receptor agonist ligand.

### BRIEF DESCRIPTION OF THE FIGURES

Figure 1: Tracking of the individual size of subcutaneous tumor nodes (surface area of the tumor lesion expressed in mm²) resulting from the intradermal inoculation of 5 x 10⁵ MC38 cells in female C57BL/6 mice.

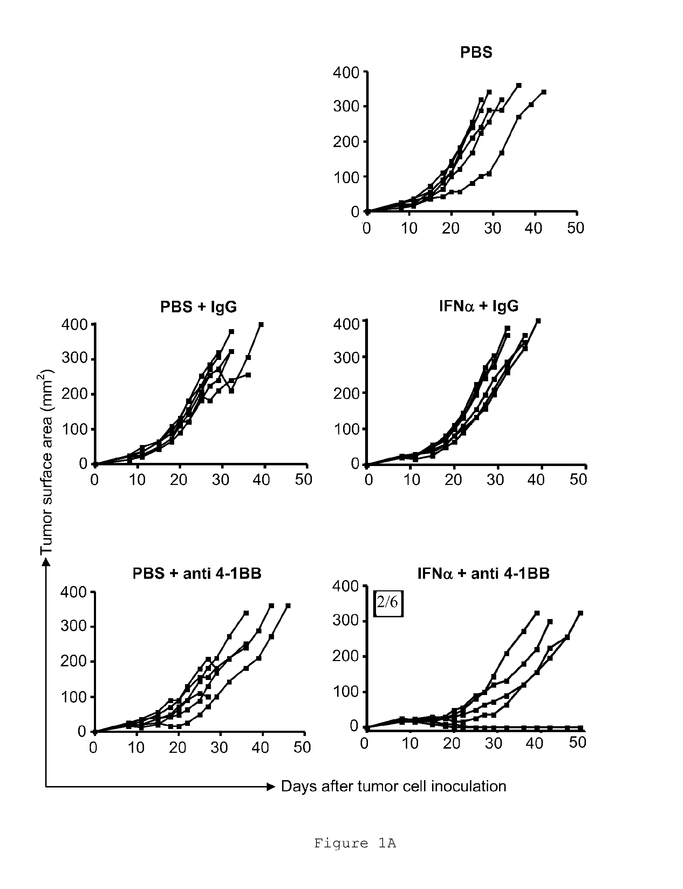

   Figure 1A shows the progression of individual tumors after being treated with two doses of different treatments applied on days 9 and 13 after the tumor cell inoculation. The following doses were administered in the different groups: 50 µl of intratumoral PBS saline solution; a polyclonal rat immunoglobulin G with an irrelevant specificity (100 µg in 100 µl intraperitoneally); the anti-mouse 4-1BB (CD137) monoclonal antibody 2A, (100 µg in 100 µl intraperitoneally); recombinant mouse IFN-α4 (0.5 x 10⁴ U intratumorally) in combination with a polyclonal rat immunoglobulin G with an irrelevant specificity (100 µg in 100 µl intraperitoneally), or the combination of recombinant mouse IFN-α4 (0.5 x 10⁴ U intratumorally) plus the administration of the anti 4-1BB antibody 2A (100 µg in 100 µl intraperitoneally). The last graph of Figure 1a shows how the combined treatment of recombinant mouse IFN-α4 plus the administration of the anti 4-1BB antibody 2A has a high therapeutic effect which is shown in the tumor-free survival of 2 out of six mice.
   Figure 1B shows the tumor surface area of each experimental group (mean ± SEM) after tumor cell inoculation.
   Figure 1C shows that the combined treatment of recombinant mouse IFN-α4 plus the administration of the anti 4-1BB antibody 2A has a higher therapeutic effect, which is shown in the tumor-free survival of 2 of out of six mice.
Figure 2: Studies in mice carrying two MC38 tumor lesions implanted simultaneously and intradermally in opposite sides of the dorsolumbar region, in a manner similar to the mice of Figure 1, but in this case in a bilateral manner.

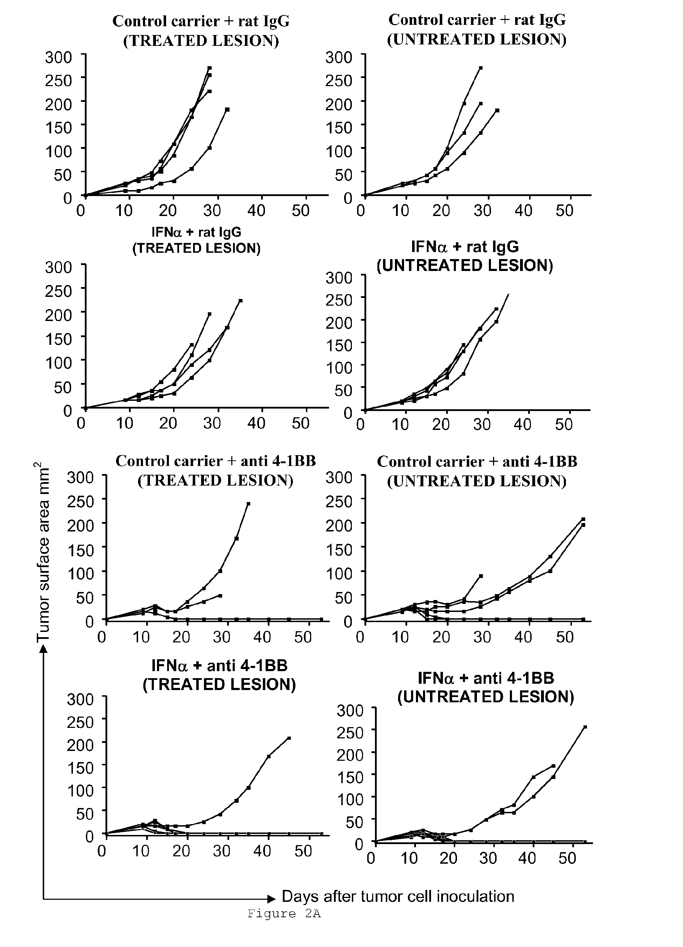

In these cases, the mice were treated with three doses applied on days 9, 12 and 15 counted from the bilateral implantation of MC38 tumor cells. The treatments consisted of the intraperitoneal injection of polyclonal rat immunoglobulin and the intratumoral injection of the carrier or medium in which mouse interferon-α is dissolved (as negative controls of the experiment) in only one of the lesions, or the administration of anti-4-1BB monoclonal antibody 2A (100 µg in 100 µl intraperitoneally) or the combinations of both treatments, always injecting interferon-α or its carrier in the corresponding control group intratumorally in only one of the nodes (right node). In the third pair of graphs of Figure 2A, the relative number of mice with complete regression of both tumor nodes in the experimental group treated with intraperitoneal anti-4-1-BB plus intratumoral control carrier was 1/5, compared to the experimental group receiving intraperitoneal anti-4-1-BB antibody plus the intratumoral injection of interferon-α (fourth pair of graphs of Figure 2A), wherein the relative number of mice with complete regressions of both tumor nodes was 4/6.

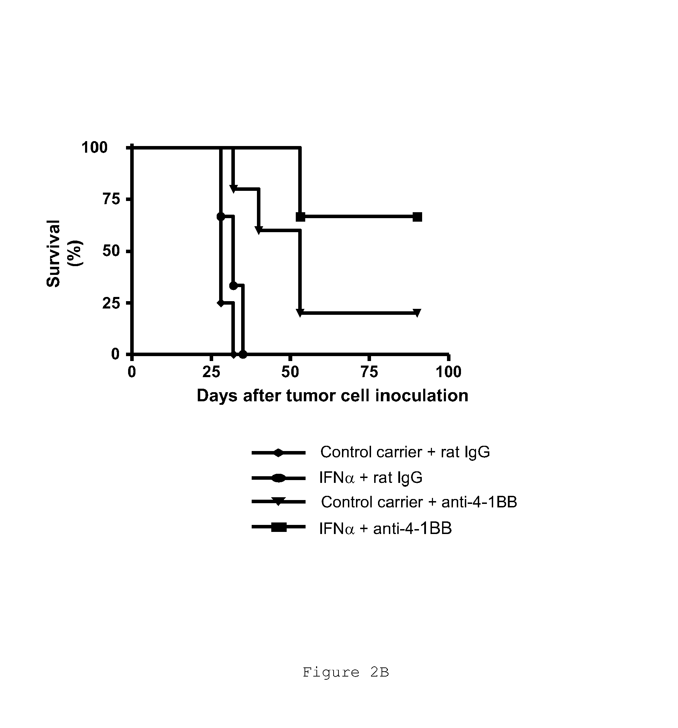

Figure 2B shows the survival of the mice after the tumor cell inoculation and after the treatment.

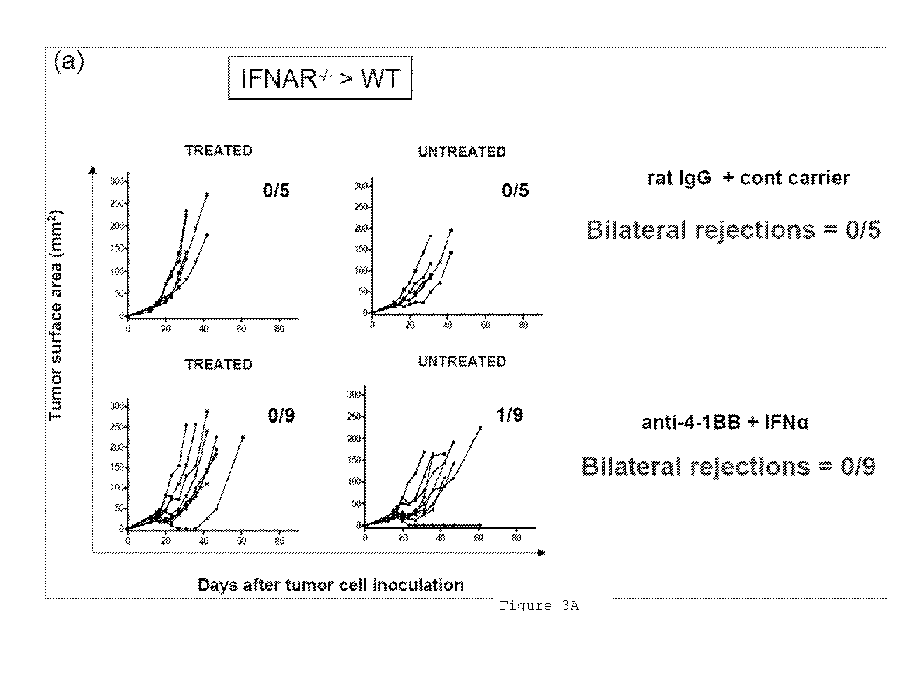

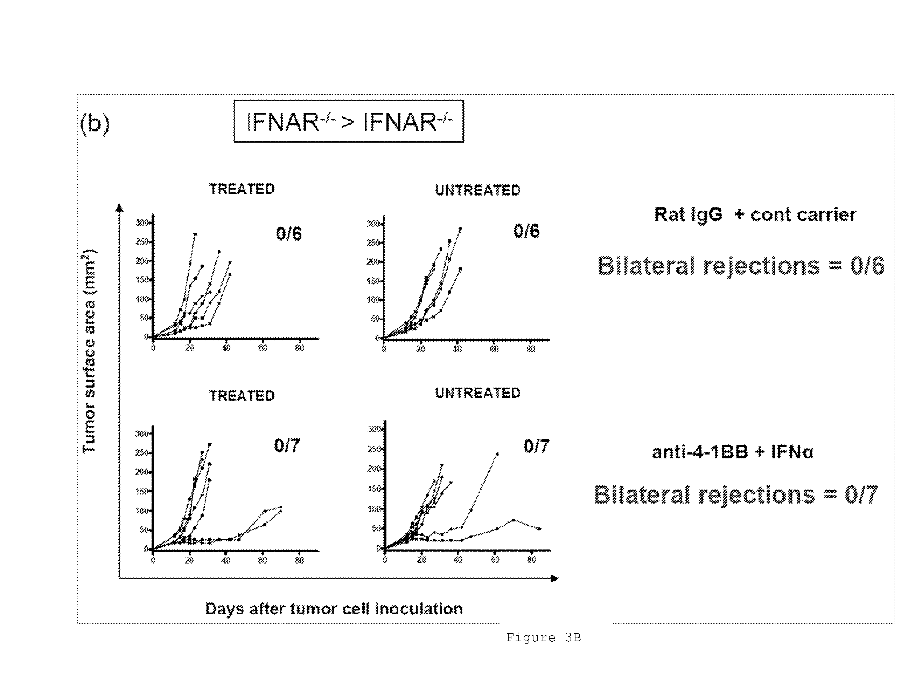

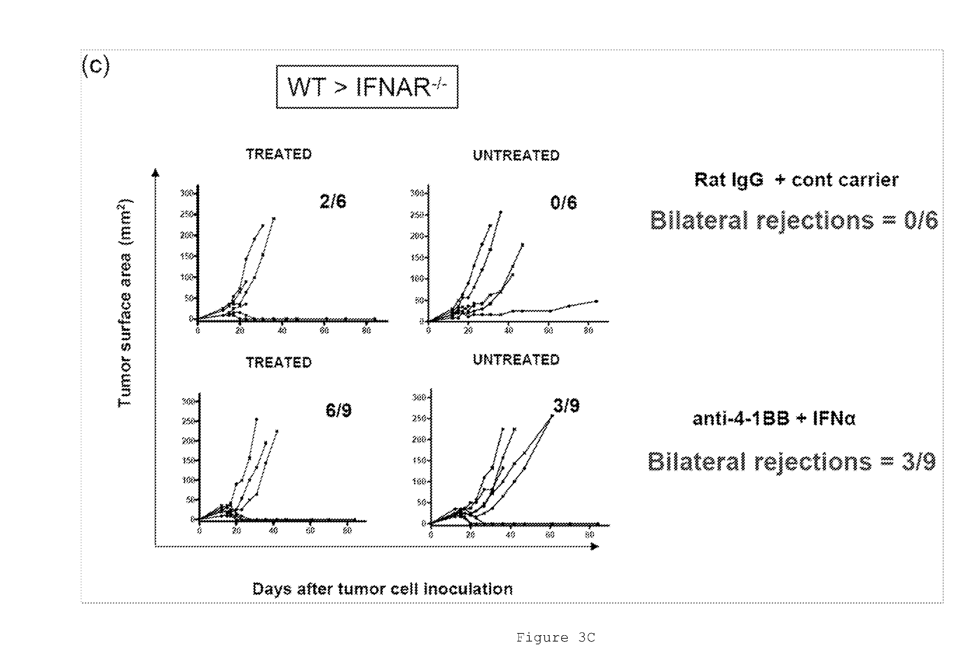

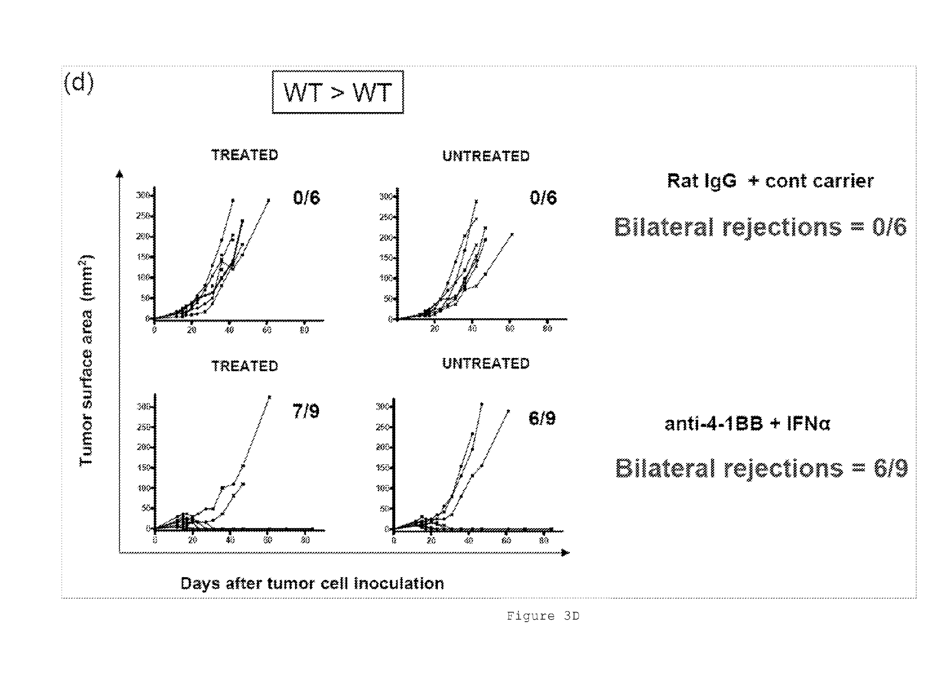

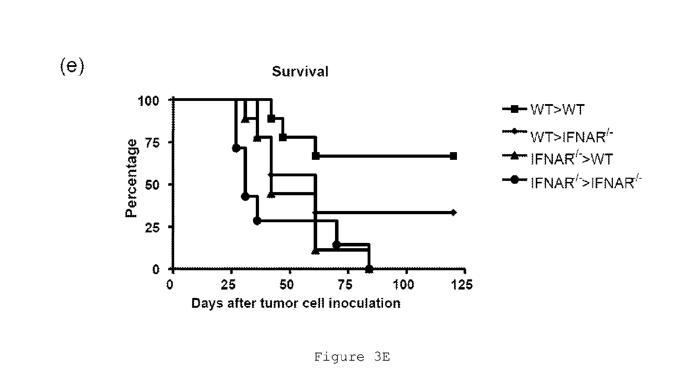

Figure 3: Tumor progression in chimeric mice bone marrow with or without expression of the interferon type I receptor in hematopoietic lineages cells.
   Groups of *wild type* C57BL/6 mice (WT) and mice deficient in the IFN type I receptor (IFNAR^{-/-}) backcrossed 12 times to the C57Bl/6 background received a lethal radiation dose of 600 rads. After 24 hours they were transplanted with 2-5x10⁶ cells of the donor mice bone marrow. The bone marrow was obtained from the femur and tibia of mice with an age of less than 3 months. The hematopoietic origin cell populations of the recipient mouse thus come from the donor mouse after a reconstitution period of 4-8 weeks. It is therefore considered to be a mouse with complete chimerism in the hematopoietic compartment. Between 6 and 8 weeks later, once the donor bone marrow has been grafted, the cell immunotherapy experiment protocols began similarly to those described in Figure 2. The chimeric mice bearing subcutaneous tumors derived from the MC38 line were treated with three doses applied on days 11, 14 and 17 after the subcutaneous implantation of tumor cells on both sides of the back. The anti-4-1BB (CD137) monoclonal antibody was injected systemically (intraperitoneally) in combination with IFN-α which was administered intratumorally in the tumor lesion on the right side. The control treatments were likewise applied as they had been used in the previously described experiments.
   Figures 3a, 3b, 3c and 3d show the progression of the size of the tumor both of the nodes treated directly with IFN-α or the control carrier and in the non-injected nodes. The experiments were conducted identically in the different types of chimera (donor-recipient combinations). The number of mice with complete regressions of both tumors in the different groups is specified in the figure.
   Figure 3e shows these results expressed as survival in the groups that had received treatment with the anti-4-1BB antibody plus IFN-α in the different types of chimeric mice.

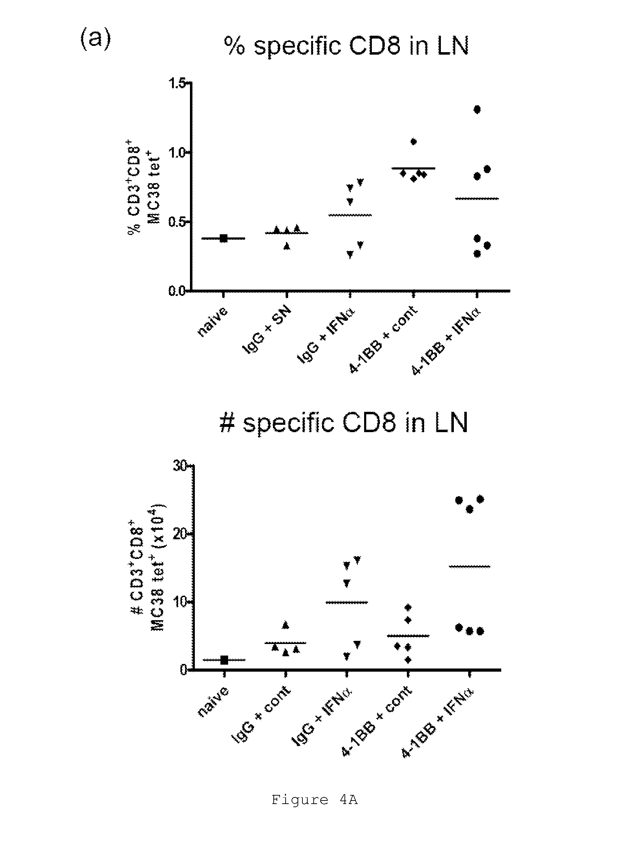

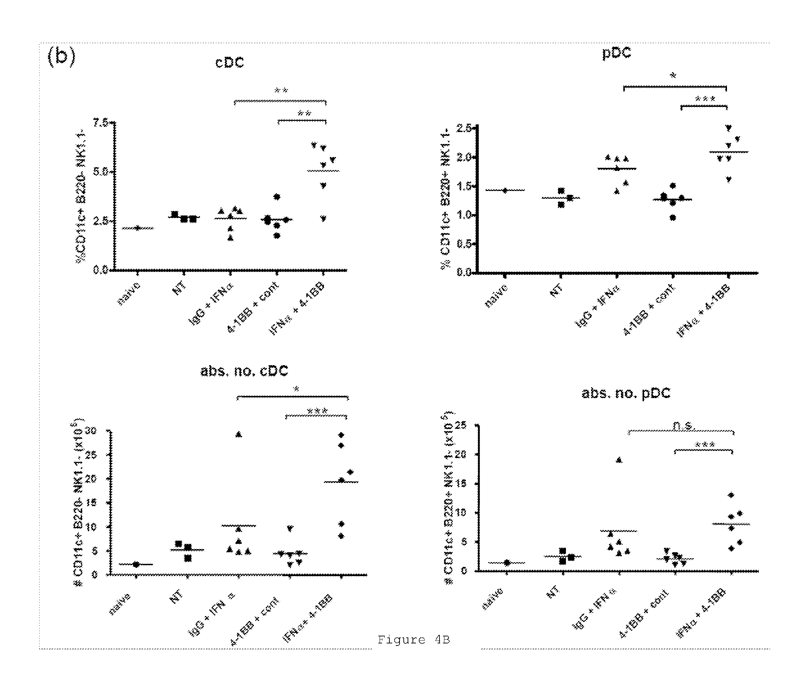

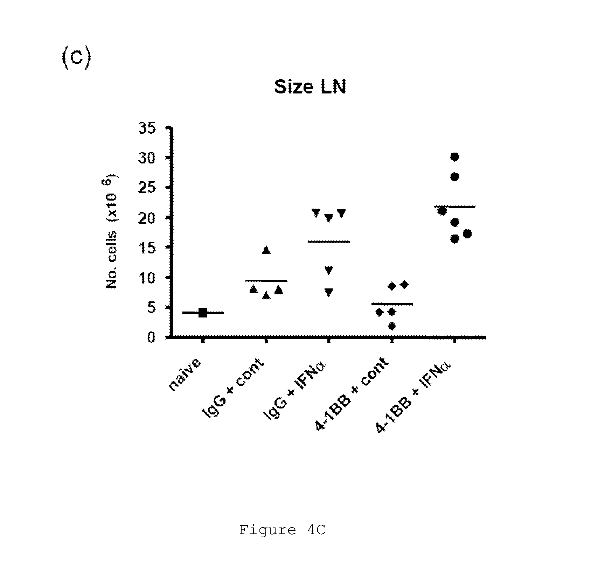

Figure 4: Analysis of the lymphocyte problem in tumor draining lymph nodes of MC38-bearing mice.
   After subcutaneously injecting MC38 tumor cells, the different treatment combinations were administered on days 9 and 12 in the same way as described for previous experiments. The mice were sacrificed 24 hours after the second dose to extract the lymph nodes from the side in which they bore the transplanted tumor. After processing the tissue from the lymph node and obtaining an aggregate-free cell suspension, immunofluorescence staining was performed for flow cytometry for the purpose of analyzing different lymphocyte populations of interest. Figure 4a shows the percentages and absolute number of tumor-specific T CD8⁺ lymphocytes in the draining nodes (LN) of the different experimental groups. These groups are defined by means of the double marking for CD8 and the binding of a set aside H2-Kb tetramer with the KSPWFTTL peptide defining a dominant antigenic epitope of the MC38 tumor. Differences were also observed in the dendritic cell population both in the conventional cells (cDC) defined as CDllc+B220-NK1.1-, and in the plasmacytoid cells (pDC) defined as CD11c^{int} B220⁺ NK1.1⁻, as shown in Figure 4b. The total number of cells of the draining nodes was further counted in this experiment, and the figures revealed important differences between the experimental groups (Figure 4c).

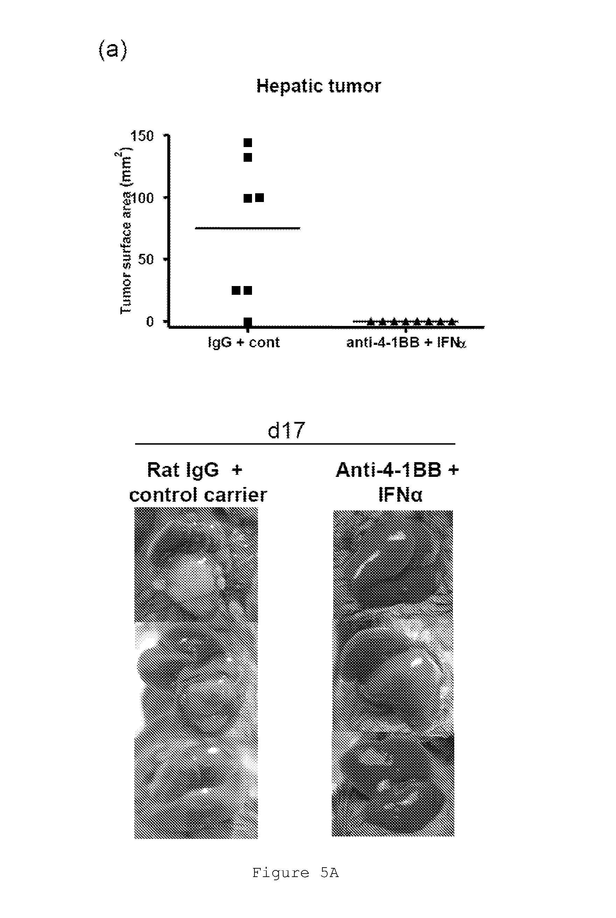

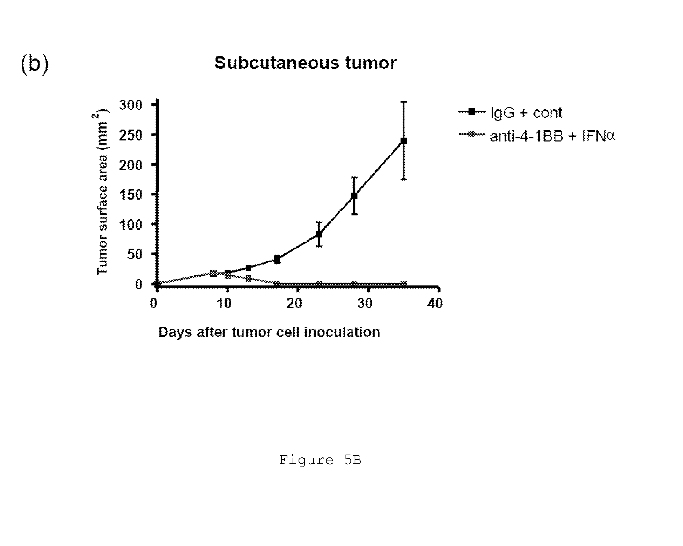

Figure 5: Treatment with intratumor IFN-α in a subcutaneous lesion and anti-4-1BB monoclonal antibodies systemically has an intense therapeutic effect against concomitant intrahepatic tumor lesions.
   In this experiment, each mouse was inoculated with MC38 tumor cells through two different ways. Firstly, they were subcutaneously injected in the same way as in the previously described experiments. Then the mice were operated on by laparotomy for the intrahepatic inoculation of the tumor cells. Treatment consisted of the intraperitoneal injection of the anti-4-1BB antibody (or polyclonal rat immunoglobulin as a control) in combination with the intratumor administration of IFN-α (or the control carrier) in the subcutaneous node on days 8, 10 and 13 after inoculation of the tumors.
   The results on the hepatic tumor were observed on day 17 after tumor inoculation by means of exploratory laparotomy (Figure 5a), including the sizes of the lesions and representative photographs of each experimental group. Progression of the size of the subcutaneous nodes is seen in Figure 5b.

### DETAILED DESCRIPTION OF THE INVENTION

The present invention relates to the treatment of cancer by means of the use of different therapeutic agents. The inventors of the present invention have found that, surprisingly, the combined administration of a 4-1BB receptor agonist ligand and a type I interferon has an effect that is clearly much higher than each treatment separately in achieving the eradication of multiple tumors formed in mice.

Without intending to be linked to any theory, it is believed that the administration of IFN-α has the capacity to induce modifications in malignant tissue or in the lymphocytes of the subject carrying the tumor, said modifications favoring the induction of an immune response which, in the case of the combined treatment, is amplified in the presence of a 4-1BB receptor agonist ligand, such as an agonist anti-4-1BB receptor antibody.

The present invention thus relates to the treatment of cancer by means of the combined administration of a 4-1BB receptor agonist ligand and a type I interferon, thus achieving a greater therapeutic effect than if said ligand and interferon are administered separately.

Therefore, in one aspect the invention relates to a composition, hereinafter, composition of the invention, comprising at least one 4-1BB receptor agonist ligand and at least one type I interferon.

In the present invention, the 4-1BB receptor agonist ligand and the type I interferon are considered to be "anti-tumor agents" or "active substances" or "active ingredients" of the composition of the invention and, therefore, said expressions are used without distinction throughout the description to refer to them.

In the present invention "4-1BB receptor agonist ligand" is understood as the ligand binding specifically to the 4-1BB receptor (also known as CD137 receptor) and which, upon binding, can stimulate some of the costimulation signals characteristic of the binding of said receptor with its natural ligand (4-1BBL or CD137-L) or other signals resulting from the binding of said receptor with an agonist 4-1BB receptor.

There is a wide variety of immunological assays available for detecting the activity of 4-1BB receptor agonist ligands, such as the *in vitro* T-cell growth costimulation described in Wilcox, R. et al. 2002, J. Clin. Invest. Vol. 109(5): 651-659. Briefly, said assay consist of placing a T-cell culture in the presence of a 4-1BB agonist ligand, for example, an anti-4-1BB monoclonal antibody, and measuring T-cell proliferation by means of incorporating tritium. It is alternatively possible to detect the agonistic activity of a 4-1BB agonist ligand by means of detecting changes in the cyclin D2 expression levels in T-cells after being exposed to said ligand, as described in W02005035584.

Examples of 4-1BB receptor agonist ligands are (i) the natural ligand of 4-1BB receptor, or a functionally equivalent variant thereof conserving the capacity to bind to 4-1BB receptor and induce costimulus signals thereupon, or (ii) an agonist antibody against the 4-1BB receptor, or a functionally equivalent variant thereof which can bind specifically to the 4-1BB receptor, and more particularly to the extracellular domain of said receptor, and induce some of the costimulation signals controlled by this receptor and associated proteins. The binding specificity can be for the human 4-1BB receptor or for a 4-1BB receptor homologous to the human receptor of a different species.

Thus, in a particular embodiment of the composition of the invention at least one 4-1BB receptor agonist ligand is the natural ligand of 4-1BB receptor or a functionally equivalent variant thereof. The human and murine natural 4-1BB receptor ligands are known in the state of the art. The human 4-1BB receptor ligand corresponds to the 254 amino acid polypeptide the sequence of which is shown in the Uniprot database with accession number P41273 and which is encoded by the polynucleotide the sequence of which is shown in the GenEMBL database with accession number U03398 (SEQ ID NO: 1). The mouse 4-1BB receptor ligand corresponds to the polypeptide the sequence of which is indicated in the Uniprot database with accession number P41274 and which is encoded by the polynucleotide the sequence of which is shown in the GenEMBL database with accession number L15435 (SEQ ID NO: 2). The 4-1BB receptor ligand is a type II membrane protein. Therefore, the invention contemplates the use of the complete protein (SEQ ID NO: 3 and 4 for the human and murine proteins, respectively) as well as the extracellular region thereof (SEQ ID NO: 5 and 6 for the human and murine proteins, respectively). The invention thus contemplates the use of a soluble form of the 4-1BB receptor ligand comprising amino acids 106 to 309 of the murine protein (as described in US 6,355,779), of fusion proteins between the soluble form of 4-1BB and the Fc region (as described in US 6,355,779), of an immunoglobulin molecule and dimeric versions thereof (as described in US 6,355,779), of trimers of the soluble fraction of the 4-1BB receptor ligand (as described in WO2007000675), of trimers of fusion proteins formed by the extracellular domain of the 4-1BB receptor ligand, a linker and the region corresponding to the variable regions of the light and heavy chains of a specific immunoglobulin for a receptor present in T-cells and able to promote the activation thereof, as have been described in W02004069876.

A functionally equivalent variant of the natural ligand of 4-1BB receptor is understood as any polypeptide the sequence of which can be obtained by means of inserting, substituting or eliminating one or more amino acids of the sequences of the natural 4-1BB ligand sequence, and which polypeptide at least partially conserves the capacity to stimulate the 4-1BB receptor, determined by means of the aforementioned assay. The variants of the natural 4-1BB ligand preferably have a sequence identity with said ligand of at least 50%, at least 60%, at least 70%, at least 80%, at least 90%, at least 91%, at least 92%, at least 93%, at least 94%, at least 95%, at least 96%, at least 97%, at least 98% or at least 99%. The degree of identity between the variants and the natural ligand is determined by using computer algorithms and methods that are widely known for the persons skilled in the art. The identity between two amino acid sequences is preferably determined by using the BLASTP algorithm (BLASTManual, Altschul, S., et al, NCBI NLM NIH Bethesda, Md. 20894, Altschul, S., et al., J. Mol. Biol. 21 5: 403-410 (1990).

In another particular embodiment of the composition of the invention, at least one 4-1BB receptor agonist ligand is an agonist anti-4-1BB receptor antibody which can be of any class or sub-class of immunoglobulins, such as IgG, IgM, IgA, IgD and IgE. In a particular embodiment, at least one of said agonist anti-4-1BB receptor antibodies is an IgG-2A type immunoglobulin.

In the present invention, the term "antibody" must be interpreted in a broad sense and includes multispecific, polyclonal, monoclonal antibodies and (F(ab')2, Fab fragments thereof, provided that they can recognize the antigen of interest, can bind specifically to the 4-1BB receptor or to the extracellular domain of said receptor.

In the present invention, "agonist 4-1BB receptor antibody" is understood as any antibody which can bind specifically to the 4-1BB receptor, or to the extracellular domain of said receptor, and induce some of the costimulation signals controlled by the 4-1BB receptor and associated proteins. Examples of antibodies which can be used in the context of the present invention are, for example and are not limited to, polyclonal antibodies, monoclonal antibodies, recombinant antibodies, chimeric antibodies, humanized antibodies, completely human antibodies, etc.

Polyclonal antibodies are originally heterogeneous mixtures of antibody molecules produced in the serum of animals which have been immunized with an antigen. They also include monospecific polyclonal antibodies obtained from heterogeneous mixtures, for example, by means of column chromatography with peptides of a single epitope of the antigen of interest.

A monoclonal antibody is a homogeneous population of specific antibodies for a single epitope of the antigen. These monoclonal antibodies can be prepared by means of conventional techniques that have already been described, for example, in Köhler and Milstein [Nature, 1975; 256:495-397] or Harlow and Lane ["Using Antibodies. A Laboratory Manual" by E. Harlow and D. Lane, Editor: Cold Spring Harbor Laboratory Press, Cold Spring Harbor, New York; 1998 (ISBN 978-0879695439)].

A chimeric antibody is a monoclonal antibody constructed by means of cloning or recombining antibodies from different animal species. In a typical but non-limiting configuration of the invention, the chimeric antibody includes a part of a monoclonal antibody, generally the variable region (Fv) including the sites for the recognition and binding to the antigen, and the other part corresponding to a human antibody, generally the part including the constant region and the adjacent constant region.

A completely human antibody is an antibody or antibodies which have been produced in transgenic animals with a human immune system or by *in vitro* immunization of human immune cells (including both genetic and traditional immunization with or without adjuvants and pure or impure antigen; or by means of any method for exposing the antigen to the immune system) or by means of native/synthetic libraries produced from human immune cells. These antibodies can be obtained and screened from transgenic animals (mice, for example) into which human immunoglobulin genes have been cloned and which are immunized with the target antigen (with the 4-1BB receptor in the present invention). These antibodies can be obtained by screening human phage-displayed single-chain variable regions (scFv) or antigen binding regions (Fab) and subsequently, cloning and grafting into a human antibody or by means of any other method, known by the persons skilled in the art, for producing and displaying the libraries generated by cloning the variable regions of both chains and subsequently cloning/mutating them to generate antibody libraries.

A humanized antibody is a monoclonal antibody constructed by means of cloning and grafting the hypervariable complementarity determining regions (CDR) of a murine monoclonal antibody into a human antibody in substitution of its own hypervariable CDR regions.

Thus, in a particular embodiment of the composition of the invention, at least one agonist anti-4-1BB receptor antibody is a humanized antibody. Examples of specific humanized antibodies for the 4-1BB receptor have been described in W0200410947.

In addition, in the context of the present invention, the term "antibody" also includes variants with an altered glycosylation pattern, as has been described in W02006088447 as well as glycosylated or non-glycosylated antibody fragments which have been obtained from the protein or by means of recombinant technology, which can consist of (i) variable antibody areas bound to one another by a binding peptide (scFv), (ii) the variable area next to the constant area CH1 of the heavy chain (Fd) bound to the light chain by means of cysteines or by means of binding peptides and disulfide bridge (scFab), (iii) new variants, such as heavy chains alone, or (iv) any modification carried out on the antibody fragments for the purpose of making them more similar, less immunogenic (humanized) or more stable in biological fluids and so that they have the capacity to produce some of the costimulus signals characteristic of the 4-1BB receptor.

The agonist 4-1BB receptor antibodies described in the present invention can be obtained by conventional recombinant or genetic engineering techniques, conventional techniques for antibody production, extraction and purification from biological fluids or tissues, or any other conventional technique for obtaining proteins and antibodies, which are widely known by persons skilled in the art. When the 4-1BB receptor agonists are antibodies, the following techniques, among others, can be used for their production, without this being any limitation whatsoever: immunization techniques in animals, including transgenic animals for human immunoglobulin genes, production of monoclonal antibodies by means of hybridomas, production by means of antibody libraries, which can be native, synthetic or derived from organisms immunized against the antigen of interest and which could be screened by means of different display methods (phage display, ribosome display, etc.) and subsequently, by means of genetic engineering techniques, could be redesigned and expressed in vectors designed for producing recombinant antibodies with different sizes, composition and structure. A review of the main methods for producing and purifying antibodies can be found in, for example:
■ "Handbook of Therapeutic Antibodies", by S. Dübel. Editor: Wiley-VCH, 2007, Vol: I to III (ISBN 978-3527314539);
■ "Antibodies: Volume 1: Production and Purification" by G. Subramanian Ed., Editor: Springer, 1st Ed, 2004 (ISBN 978-0306482458);
■ "Antibodies: Volume 2: Novel Technologies and Therapeutic Use", by G. Subramanian Ed., Editor: Springer, first edition, 2004 (ISBN 978-0306483158);
■ "Molecular Cloning: a Laboratory manual", of J. Sambrook and D.W. Russel Eds., Publisher: Cold Spring Harbor Laboratory Press, third edition, 2001 (ISBN 978-0879695774).

More specifically, any of the methods described in WO98/16249, W02004/010947, US2004/0109847 and US2005/0013811, the entire contents of which are included as a reference, can be used to produce and obtain antibodies which bind specifically to the 4-1BB receptor.

As has been indicated above, the composition of the invention comprises at least one 4-1BB receptor agonist ligand and one type I interferon. At least one interferon of those used in the preparation of the medicament of the invention is any class of type I interferon, such as IFN-α, IFN-β, IFN-δ, IFN-ε, IFN-κ, IFN-τ and IFN-ω.

In a particular embodiment, at least one type I interferon comprised in the composition of the invention is selected from the group comprising interferon-alpha (IFN-α) and interferon-beta (IFN-β).

When the type I interferon is IFN-α, it can correspond to any interferon encoded by any gene that is a member of the human IFN-α gene family. In a particular embodiment, at least one type I interferon is an IFN-α selected from the group of IFN-α2a, IFN-α2b, IFN-α4, IFN-α5, IFN-α8 and combinations thereof, including their combination with other substances in pharmaceutical formulations.

A list of type I interferon varieties, particularly IFN-α and IFN-β which can be used according to the invention, can be found in Bekisz et al. (Growth Factors, 2004; 22:243-251) and in Petska et al. (Immunological Reviews, 2004; 202:8-32). A combination of interferons, such as IFN-αn1 (lymphoblastoid derivative) or IFN-α3 [combination of interferons produced by human leukocytes stimulated with the Sendai virus (or another virus) or viral particles], for example, could additionally be used to prepare the composition of the invention.

The origin of the type I interferon used does not form a critical aspect of the invention. It can have a natural origin, extracted and purified from biological fluids or tissues, or it can be produced by means of conventional recombinant or genetic engineering methods, such as those described in Sambrook and Russel ("Molecular Cloning: a Laboratory manual" by J. Sambrook, D.W. Russel Eds. 2001, third edition, Cold Spring Harbor, New York), by synthesis processes or any another conventional technique described in the state of the art.

In a particular embodiment of the invention, at least one type I interferon comprised in the composition of the invention is in a pegylated form. Some examples for preparing pegylated forms of interferon can be found in US 5,762,923 and US 5,766,582. In addition, it is also possible to use some of the pegylated or non-pegylated forms of interferon that are already commercially available. These include, but are not limited to, ROFERON-A (recombinant human IFN-α2a) and PEGASYS (pegylated IFN-α) of Hoffmann La Roche Inc., INTRON-A (human recombinant IFN-α2b) and PEG-INTRON (pegylated IFN-α2b) of Schering Corp., ALFERON-N (IFN-α3n, combination of natural interferons) of Interferon Sciences, or IFNERGEN (IFN-αcon1) of InterMune Pharmaceuticals Inc., the sequence of which is a consensus sequence that does not exactly correspond to a natural sequence. IFN-β formulations, such as AVONEX (IFN-β1a) of Biogen Idec, REBIF (IFN-β1a) of EMD Serono, Inc, and BETASERON (IFN-β1b) of Bayer Health Care, for example, are also included.

In another particular embodiment, the composition of the invention further comprises a chemotherapeutic compound.

In the present invention, "chemotherapeutic compound" is understood as those compounds or agents used in the treatment of cancer, such as alkylating agents, alkaloids, antimetabolites, anti-tumor antibodies, nitrosoureas, analog antagonists/agonists, immunomodulators, enzymes and others. Tables 1 and 2 of patent application W02004/093831 describe useful chemotherapeutic agents for implementing the present invention.

According to the present invention, the combined administration of a 4-1BB receptor agonist ligand and a type I interferon in the treatment of cancer has a greater therapeutic effect than if said ligand and interferon are administered separately.

Therefore, in another aspect, the invention relates to a composition according to the present invention for its use in medicine.

In another aspect, the invention relates to the use of a composition according to the present invention for preparing a medicament for the treatment or the prevention of cancer.

In another aspect, the invention relates to a composition according to the present invention for the treatment or the prevention of cancer.

In the context of the invention, "anti-tumor treatment", "treatment of cancer" or "prevention of cancer" is understood as the combined administration of a 4-1BB receptor agonist ligand and of type I interferon to prevent or delay the onset of symptoms, complications or biochemical indications of the cancer or tumor, to alleviate its symptoms or to stop or inhibit its development and progression, such as the onset of metastases, for example. The treatment can be a prophylactic treatment to delay the onset of the disease or to prevent the manifestation of its clinical or subclinical symptoms, or a therapeutic treatment to eliminate or alleviate the symptoms after the manifestation of the disease or in relation to its surgical or radiotherapy treatment.

The cancer to be treated with the composition of the invention can be any type of cancer or tumor. These tumors or cancer include, and are not limited to, hematological cancers (leukemias or lymphomas, for example), neurological tumors (astrocytomas or glioblastomas, for example), melanoma, breast cancer, lung cancer, head and neck cancer, gastrointestinal tumors (stomach, pancreas or colon cancer, for example), liver cancer, renal cell cancer, genitourinary tumors (ovarian cancer, vaginal cancer, cervical cancer, bladder cancer, testicular cancer, prostate cancer, for example), bone tumors and vascular tumors.

Thus, in a particular embodiment, the cancer to be treated or prevented by using the composition of the invention or the medicament prepared from said composition is a solid tumor or, in another particular embodiment, it is a colon carcinoma.

The composition of the invention can be administered by different methods, intravenously, intraperitoneally, subcutaneously, intramuscularly, topically, intradermally, intranasally or intrabronchially, for example, and can be administered locally or systemically or directly to the target site. A review of the different methods of administration of active ingredients, of the excipients to be used and of the processes for manufacturing them can be found in Tratado de Farmacia Galénica, C. Faulí i Trillo, Luzán 5, S.A. de Ediciones, 1993 and in Remington's Pharmaceutical Sciences (A.R. Gennaro, Ed.), 20th edition, Williams & Wilkins PA, USA (2000).

The dosage regimen will be determined by the doctor and the clinical factors. As is well known in medicine, dosages depend on many factors including the physical characteristics of the patient (age, size, sex), the method of administration used, the severity of the disease, the particular compound used and the pharmacokinetic properties of the individual.

The composition of the invention can contain an amount of anti-tumor agents which can vary within a wide range, but always in therapeutically effective amounts.

In the present invention, a "therapeutically effective amount" is understood as the amount of a 4-1BB receptor agonist ligand and of type I interferon that is enough to cause a delay in tumor growth or the inhibition thereof, or to induce an increase of the anti-tumor immune response.

The composition of the invention can thus contain an amount of anti-tumor agents ranging between 0.1 and 2,000 mg, preferably in the range of 0.5 to 500 mg and, even more preferably between 1 and 200 mg. Suitable doses of the compositions can range between 0.01 and 100 mg/kg of body weight, preferably between 0.1 to 50 mg/kg of body weight, more preferably, between 0.5 and 20 mg/kg of body weight. The composition can be administered a variable number of times a day, particularly from 1 to 4 doses a day.

The composition according to the present invention has proved to be useful in anti-tumor treatment. Therefore, in another aspect, the invention relates to a pharmaceutical preparation comprising the composition of the invention and a pharmaceutically acceptable carrier.

For use in medicine, the combinations of compounds of the invention can be in the form of a prodrug, salt, solvate or clathrate, either in isolated form or in combination with additional active agents. The combinations of compounds according to the present invention can be formulated together with an excipient which is acceptable from the pharmaceutical point of view. Preferred excipients for their use in the present invention include sugars, starches, celluloses, gums and proteins. In a particular embodiment, the pharmaceutical composition of the invention will be formulated in a solid (for example, tablets, capsules, sugar-coated tablets, granules, suppositories, crystalline or amorphous sterile solids which can be reconstituted to provide liquid forms, etc.), liquid (for example, solutions, suspensions, emulsions, elixirs, lotions, unguents etc.) or semisolid (gels, ointments, creams and the like) pharmaceutical dosage form. The pharmaceutical compositions of the invention can be administered by any method, including and not limited to oral, intravenous, intramuscular, intra-arterial, intramedullary, intrathecal, intraventricular, transdermal, subcutaneous, intraperitoneal, intranasal, enteric, topical, sublingual or rectal administration. A review of the different forms of administration of active ingredients, of the excipients to be use and of the processes for manufacturing them can be found in Tratado de Farmacia Galénica, C. Faulí i Trillo, Luzán 5, S.A. de Ediciones, 1993 and in Remington's Pharmaceutical Sciences (A.R. Gennaro, Ed.), 20th edition, Williams & Wilkins PA, USA (2000) Examples of pharmaceutically acceptable carriers are known in the state of the art and include phosphate buffered saline solutions, water, emulsions such as oil/water emulsions, different types of wetting agents, sterile solutions, etc. The compositions comprising said carriers can be formulated by conventional processes known in the state of the art.

In another aspect, the invention relates to a kit, hereinafter kit of the invention, comprising one or several containers of
(i) a pharmaceutically acceptable formulation of at least one 4-1BB receptor agonist ligand or a functionally equivalent variant thereof
(ii) a pharmaceutically acceptable formulation of at least one type I interferon or a functionally equivalent variant thereof and, optionally,
(iii) a pharmaceutically acceptable formulation of a chemotherapeutic compound.

In the present invention, a "kit" is understood as a product containing the different active ingredients forming the composition packed so as to allow their transport, storage and their simultaneous or sequential administration. The kits of the invention can thus contain one or more suspensions, tablets, capsules, inhalers, syringes, patches and the like, containing the active ingredients of the invention and which can be prepared in a single dose or as multiple doses. The kit can additionally contain a suitable carrier for resuspending the compositions of the invention such as aqueous media, such as saline solution, Ringer's solution, lactated Ringer's solution, dextrose and sodium chloride, water-soluble media, such as alcohol, polyethylene glycol, propylethylene glycol and water-insoluble carriers such as corn oil, cottonseed oil, peanut oil, sesame oil, ethyl oleate, isopropyl myristate and benzyl benzoate. Another component which can be present in the kit is a packing which allows maintaining the formulations of the invention within determined limits. Suitable materials for preparing such packings include glass, plastic (polyethylene, polypropylene, polycarbonate and the like), bottles, vials, paper, sachets and the like.

In a particular embodiment of the kit of the invention, at least one 4-1BB receptor agonist ligand is the natural ligand of 4-1BB receptor.

In another particular embodiment of the kit of the invention, at least one 4-1BB receptor agonist ligand is an agonist anti-4-1BB receptor antibody which, in an even more particular embodiment, is an IgG-2A type immunoglobulin or a humanized antibody.

In another particular embodiment of the kit of the invention, at least one type I interferon is selected from the group comprising IFN-α and IFN-β.

In another particular embodiment of the kit of the invention, at least one type I interferon is an IFN-α selected from the group of IFN-α2a, IFN-α2b, IFN-α4, IFN-α5, IFN-α8 and combinations thereof.

In another particular embodiment of the kit of the invention, at least one type I interferon is a pegylated interferon.

The different uses of the kit of the invention form additional aspects thereof. Thus, in one aspect the invention relates to a kit according to the present invention for its use in medicine.

In another aspect, the invention relates to a kit according to the present invention for its use in the treatment or prevention of cancer.

In another aspect, the invention relates to the use of a kit according to the invention in the preparation of a medicament for the treatment or the prevention of cancer. In a particular embodiment, said cancer is a solid tumor or a colon carcinoma.

The kit of the invention can additionally contain instructions for the simultaneous, sequential or separate administration of the different pharmaceutical formulations present in the kit. Therefore, in a particular embodiment, the kit of the invention further comprises instructions for the simultaneous, sequential or separate administration of the different components.

Said instructions can be found in the form of printed material or in the form of an electronic support which can store instructions such that they can be read by a subject, such as electronic storage media (magnetic disks, tapes and the like), optical media (CD-ROM, DVD) and the like. The media can additionally or alternatively contain Internet websites providing said instructions.

As persons skilled in the art understand, the pharmaceutical formulations of the kit of the invention, i.e., the pharmaceutically acceptable formulations comprising at least one 4-1BB receptor agonist ligand or at least one type I interferon or a chemotherapeutic agent will be formulated according to the method of administration to be used. Thus, in a particular embodiment, the pharmaceutical formulation comprising at least one 4-1BB receptor agonist ligand will be formulated in a suitable manner for its systemic administration, and the pharmaceutical formulation comprising at least one type I interferon will be formulated in a suitable manner for its intratumoral administration. Additionally, in the context of the present invention, the pharmaceutical formulation comprising type I interferon will preferably be formulated in a suitable manner favoring its continuance in the administered site, such as the tumor tissue, or delaying its elimination therefrom.

As indicated above in this description and without intending to be linked to any theory, it is proposed that the intratumoral administration of a type I interferon induces modifications in the malignant tissue which favor the induction of an immune response which, in the case of the combined treatment, is amplified in the presence of the 4-1BB receptor agonist ligand. Therefore, in the context of the present invention, the 4-1BB receptor agonist ligand is preferably administered parenterally or systemically and the type I interferon is administered intratumorally.

Therefore, in another aspect, the invention relates to the use of a type I interferon, or a functionally equivalent variant thereof, for promoting the anti-tumor effect of a 4-1BB receptor agonist ligand.

As persons skilled in the art understand, instead of the combined administration of a 4-1BB receptor agonist ligand and a type I interferon for the treatment of cancer, another way of implementing the present invention consists of administering a vector comprising the nucleotide sequences encoding a 4-1BB receptor agonist ligand and a type I interferon. When the vector is expressed in the receptor organism, it will thus produce the corresponding proteins carrying out the aforementioned therapeutic effect for the treatment of cancer.

Therefore, in another aspect, the invention relates to a polynucleotide, hereinafter polynucleotide of the invention, comprising
(i) a nucleotide sequence encoding a 4-1BB receptor agonist ligand or a functionally equivalent variant thereof, and
(ii) a nucleotide sequence encoding a type I interferon or a functionally equivalent variant thereof,
   wherein both sequences are preceded by expression regulating sequences.

The definition of "functionally equivalent variant" coincides with the definition provided above in relation to the compositions containing the polypeptide corresponding to the 4-1BB ligand.

Persons skilled in the art understand that mutations in the nucleotide sequence of the 4-1BB receptor agonist ligand which give rise to conservative substitutions of amino acids in non-critical positions for the functionality of the protein, are evolutionally neutral mutations that do not affect its overall structure or its functionality. Therefore, the term "functionally equivalent variants" also includes (ii) variants of a 4-1BB receptor agonist ligand obtained from the amino acid sequence of a 4-1BB receptor agonist ligand by means of substituting, deleting or inserting one or more amino acids and (ii) substantially maintaining the function of the original protein.

In addition, the expression regulating sequences preceding the nucleotide sequences of the polynucleotide of the invention are operatively bound to said nucleotide sequences. As used in this description, the expression "operatively bound" means that the nucleotide sequences are within the correct reading frame for their expression under the control of said regulating sequences.

The regulating sequences useful for the present invention can be nuclear promoting sequences or, alternatively, enhancer sequences and/or other regulating sequences increasing the expression of the heterologous nucleic acid sequence. The promoter can be constitutive or inducible. If the constant expression of the heterologous nucleic acid sequence is desired, then a constitutive promoter is used. Examples of well known constitutive promoters include the cytomegalovirus (CMV) immediate-early promoter, the Rous sarcoma virus promoter, and the like. A number of other examples of constitutive promoters are well known in the art and can be used in implementing the invention. If the controlled expression of the heterologous nucleic acid sequence is desired, then an inducible promoter must be used. In a non-induced state, the inducible promoter must be "silent". "Silent" is understood to mean that in the absence of an inducer, little or no expression of the heterologous nucleic acid sequence is detected; in the presence of an inducer, however, the expression of the heterologous nucleic acid sequence occurs. The expression level can frequently be controlled by varying the concentration of the inducer. By controlling the expression, for example, by varying the concentration of the inducer such that an inducible promoter is stimulated more strongly or weakly, the concentration of the transcribed product of the heterologous nucleic acid sequence can be affected. In the event that the heterologous nucleic acid sequence encodes a gene, the synthesized amount of protein can be controlled. It is thus possible to vary the concentration of the therapeutic product. Examples of well known inducible promoters are: an androgen or estrogen promoter, a metallothionein promoter, or a promoter responding to ecdysone. Other various examples are well known in the art and can be used in implementing the invention. In addition to constitutive and inducible promoters (which usually work in a great variety of cell or tissue types), tissue-specific promoters can be used to reach the expression of the specific heterologous nucleic acid sequence in cells or tissues. Well known examples of tissue-specific promoters include several muscle-specific promoters including: skeletal α-actin promoter, cardiac actin promoter, skeletal troponin C promoter, slow contraction cardiac troponin C promoter and the creatine kinase promoter/enhancer. There are a number of muscle-specific promoters which are well known in the art and can be used in implementing the invention (for a review of muscle-specific promoters see Miller et al., (1993) Bioessays 15: 191-196).

In a particular embodiment, the polynucleotide of the invention encodes the natural ligand of 4-1BB receptor.

In another particular embodiment, the polynucleotide of the invention encodes an agonist anti-4-1BB receptor antibody which, in another more particular embodiment, said agonist anti-4-1BB receptor antibody is an IgG-2A type immunoglobulin.

In another particular embodiment, the polynucleotide of the invention encodes a humanized agonist anti-4-1BB receptor antibody.

In another particular embodiment, the polynucleotide of the invention encodes a type I interferon selected from the group comprising interferon-alpha and interferon-beta.

In another particular embodiment of the polynucleotide of the invention, the type I interferon is an interferon-alpha selected from the group of IFN-α2a, IFN-α2b, IFN-α4, IFN-α5, IFN-α8 and combinations thereof.

In another particular embodiment of the polynucleotide of the invention, the type I interferon is a pegylated interferon.

The polynucleotide of the invention can be contained within a suitable vector for its cloning into a host cell. Therefore, in another aspect, the invention relates to a vector, hereinafter vector of the invention, comprising a polynucleotide according to the present invention.

The choice of the vector will depend on the host cell in which is to be introduced. By way of example, the vector of the invention can be a plasmid or a vector which, when it is introduced in the host cell, is or is not integrated in the genome of said cell. Said vector can be obtained by conventional methods known by persons skilled in the art and can be found in, for example, Sambrock et al., 2001. "Molecular cloning: a Laboratory Manual", 3rd ed., Cold Spring Harbor Laboratory Press, N.Y., Vol. 1-3.

Nevertheless, in the scope of the present invention, the vector of the invention is preferably a suitable viral or non-viral vector for its use in gene therapy; by way of a non-limiting illustration, said vectors can be viral vectors based on retroviruses, adenoviruses, etc., or in case of non-viral vectors, the vectors can be DNA-liposome, DNA-polymer, DNA-polymer-liposome complexes, etc. [see "Nonviral Vectors for Gene Therapy", edited by Huang, Hung and Wagner, Academic Press (1999)]. Said viral and non-viral vectors containing the polynucleotide of the invention can be directly administered to the human or animal body by conventional methods. Said vectors can alternatively be used to transform, transfect or infect cells, mammal, including human, cells for example, ex vivo, and, subsequently implant them in the human or animal body to obtain the desired therapeutic effect. For their administration to the subject, said cells will be formulated in a suitable medium which does not adversely affect their viability.

Likewise, as persons skilled in the art will understand, said vector can contain, among others, multiple cloning sites, expression regulating sequences, suitable replication origins for the host cell in which the vector is to be introduced, selection markers, etc.

In another aspect, the invention relates to a cell comprising the vector of the invention.

As explained above, another way of implementing the present invention, consist of administering a vector comprising the nucleotide sequences encoding a 4-1BB receptor agonist ligand and a type I interferon. Thus, when the vector is expressed in the receptor organism, it will produce the corresponding proteins which will carry out the aforementioned therapeutic effect for the treatment of cancer.

Therefore, in another aspect the invention relates to the polynucleotide of the invention, to the vector of the invention or to the cell of the invention for their use in medicine.

In another aspect, the invention relates to the use of the polynucleotide of the invention, of the vector of the invention or of the cell of the invention in the preparation of a medicament for the treatment or the prevention of cancer.

In a particular embodiment, the cancer is a solid tumor or a colon carcinoma.

In another aspect, the invention relates to a pharmaceutical preparation comprising the polynucleotide of the invention, to the vector of the invention or to the cell of the invention and a pharmaceutically acceptable carrier.

In another aspect, the invention relates to a kit comprising the polynucleotide of the invention, the vector of the invention or the cell of the invention.

In another aspect, the invention relates to a kit comprising the polynucleotide of the invention, the vector of the invention or the cell of the invention for their use in medicine.

In another aspect, the invention relates to the use of a kit comprising the polynucleotide of the invention, the vector of the invention or the cell of the invention for preparing a medicament in the treatment or prevention of cancer.

In a particular embodiment, the use of a kit comprising the polynucleotide of the invention, the vector of the invention or the cell of the invention for preparing a medicament is aimed at the treatment or prevention of a solid tumor or a colon carcinoma.

Finally, in another aspect the invention relates to the use of a polynucleotide encoding a type I interferon, or a functionally equivalent variant thereof for promoting the anti-tumor effect of a 4-1BB receptor agonist ligand.

The invention additionally relates to a method for the treatment or the prevention of cancer comprising (i) the combined administration, in a therapeutically effective amount, of a 4-1BB receptor agonist ligand and a type I interferon together with, optionally, a chemotherapeutic compound, or (ii) the administration of a polynucleotide, a vector or a cell according to the present invention. Said method can comprise the separate, sequential or simultaneous administration of said ligand and interferon, which allows using different methods of administration for each component.

The following example illustrates the invention and must not be considered as limiting on the scope thereof.

### EXAMPLE

### Delay of tumor growth in mice by means of the combined administration of a 4-1BB receptor agonist ligand and a type I interferon

### I. MATERIALS AND METHODS

### 1.1 Cell cultures

The MC38 line obtained from James Mulé's laboratory was originally cultured *in vitro* in RPMI1640 medium (GIBCO) supplemented with 10% v/v of heat-inactivated fetal calf serum (GIBCO), 50 µg/mL of 2-mercaptoethanol, 100 U/mL of penicillin and 100 µg/mL of streptomycin. The cells are adherent and they were therefore detached from the culture flasks (GREINER) by means of incubating for 5 minutes with a trypsin solution (GIBCO) at room temperature. After the cells were washed, they were divided for culturing or were resuspended in saline serum for their injection. The number of cells was determined by means of microscopy in Neubauer chambers.

### 1.2 Tumor Cell Inoculation

5x10⁵ MC38 tumor cells were inoculated in the experimental animals by means of an insulin syringe with a 28G needle applied intradermally, and with a subcutaneous path that is enough to prevent the leakage of the cell suspension. A Hamilton #710 RN syringe (100µL) was used for the intrahepatic inoculation. The animals were kept anesthetized by means of anesthesia inhalation equipment with isoflurane during the laparoscopy. In this case the cells were injected in the left lobe of the liver of the mice.

### 1.3 Obtaining the experimental animals

The mice which are syngeneic with respect to the C57BL/6 tumor cell line (6-9-week-old females) were in an animal facility free of specific pathogens under veterinary supervision. The tumor nodes implanted in the mice were tracked and monitored by means of the measurement with a digital gage, and the tumor area was calculated by means of multiplying two perpendicular diameters expressed in millimeters. The IFNAR-/- mice are marketed by Jackson and crossed in our institution's animal house.

### 1.4 Obtaining the anti-tumor agents

### 1.4.1 Anti-4-1BB Monoclonal Antibody

The anti-4-1BB monoclonal antibody was produced by the hybridoma 2A (subclass of immunoglobulin IgG2A) obtained by Dr. Lieping Chen in the Mayo Clinic (Rochester, Minnesota, United States), which recognizes the mouse 4-1BB receptor and causes agonist effects thereupon (Wilcox RA, et al. 2002. J Clin Invest. 109(5):651-9).

The antibody was purified from the supernatant of the hybridoma cell culture by means of affinity chromatography on columns packed with protein G sepharose according to the manufacturer's instructions (Pharmacia). The antibody in solution was dialyzed against phosphate buffered saline (PBS), and the concentration of the antibody was determined by means of spectrometry analyzing the absorbance of the antibody solution at 280 nM compared to the saline buffer. The antibody was stored at -80°C until its use after verifying its antigen binding capacity on activated mouse T-cells and determining the absence of contaminating endotoxin. The control antibody used is a polyclonal mouse immunoglobulin G produced by SIGMA and stored in a similar manner.

### 1.4.2 Type I interferon

The mouse IFN-α4 was produced in a hybridoma line stably transfected with an expression plasmid (Le Bon A. et al. 2003. Nature Immunology, Vol. 4:1009-1015). The gene encoding IFN-α4 was cloned into the pEE12 expression plasmid (Celltech). After its amplification in *Escherichia coli,* the plasmid was purified and the transgene was sequenced. The pEE12 plasmid encoding IFN-α4 cDNA was used to transfect the NSo mouse myeloma cell line. The colonies (clones) were subjected to screening after growth in screening culture medium and a single colony of transfecting cells was chosen due to its high expression of IFN-α4. The cells were cultured for 10-15 days at a density of 0.5x10⁶ cells/ml in serum-free medium supplemented with Cholesterol Lipid Concentrate (1x; Life Technologies). The IFN-α4 content of the supernatant of the collected culture was analyzed by means of an assay of the cytopathic effect inhibition of the vesicular stomatitis virus on L-cells (ATCC Number: CCL-1) cultured in a monolayer in Falcon microplates (Becton Dickinson). The IFN-α4 preparations showed an activity of 2x10⁶ U/ml. The concentration of IFN-α was verified by means of IFN-α enzyme-linked immunosorbent assay (ELISA) kit (PBL Biomedical Laboratories).

A culture medium which was identical but in which the transfectants used have not proliferated was used as a control (control carrier). The IFN-α4 was quantified by means of ELISA compared to a standard line (R&D)

### 1.5 Administration of the anti-tumor agents

The antibodies were injected by intraperitoneal puncture and injection of the solution containing the antibody into the peritoneal cavity. The mouse IFN-α4 or the control carrier was injected into the tumor node which is accessed by means of a 28G insulin syringe.

### 1.6 Obtaining the cell suspension from lymph nodes

The tumor drainage lymph nodes (inguinal lymph nodes), on the same side where the latter was inoculated, were incubated in collagenase/DNase (Roche, Basel, Switzerland) for 15 minutes at 37°C and mechanically disintegrated before being passed through a sterile, 70 µm pore size nylon mesh (BD Falcon). The cell suspension was stained by means of direct immunofluorescence with combinations of several monoclonal antibodies conjugated to different fluorochromes and analyzed by flow cytometry (FACS SCAllibur. BD Biosciences).

### 1.7 Antibodies and flow cytometry

The cells isolated from the lymph nodes (10⁶ per sample) were pretreated with anti-CD16/32 (2.4G2 clone; BD Biosciences-Pharmingen) to reduce non-specific binding by binding to immunoglobulin FC receptors. The following monoclonal antibodies were used for FACS staining: anti-CD8a-fluorescein isothiocyanate (FITC) (53-6.7; eBioscience), anti-CD3e allophycocyanin (APC) (145-2C11; BD Biosciences-Pharmingen), anti-CD11c APC (N418; eBioscience), anti-B220 FITC (RA3-6B2; BD Biosciences-Pharmingen), anti-NK1.1 phycoerythrin (PE) (12-5941; eBioscience). The cells were marked with the tetramer iTAg MHC Class I loaded with the MC38 KSPWFTTL peptide and conjugated with (PE) (Beckman Coulter) to identify tumor-specific CD8 lymphocytes. A FACSCalibur flow cytometer (BD Biosciences) was used to acquire and analyze the samples.

### II. RESULTS

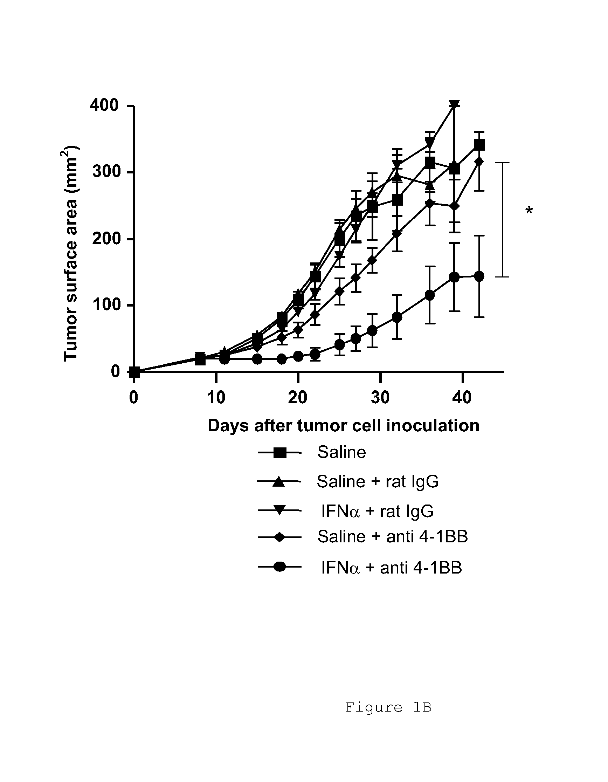

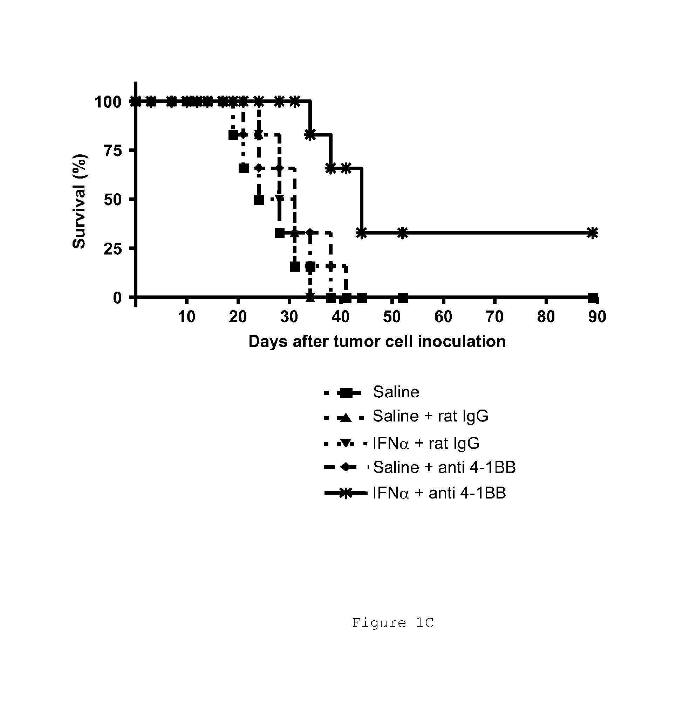

As observed in the results shown in Figure 1, the repeated administration of the anti-4-1-BB antibody 2A slightly delays MC38-derived tumor growth. In a group of 6 syngeneic mice treated intraperitoneally with 100 µg of antibody (standard dose in the literature) on days 9, 13 after the implantation of tumor cells, tumor regression of MC38-derived tumors was observed. In contrast, the repeated administration of IFN-α4 (10⁴ U/dose) into the tumor node on day 9 and 12 in a similar group of mice, does not alter the tumor progression compared to the control groups. However, when a group of mice receives these treatments in a combined manner, the anti-tumor effect is shown, the tumor growth being delayed, which tumors are reduced until finally disappearing in two out of six cases.

To check if the anti-tumor effect is systemic, i.e., if the effect is carried out at a distance on tumor nodes that are not treated intratumorally with type I interferon, two subcutaneous MC38 cell-derived nodes located a distance away were implanted on both sides of the mouse (Figure 2). In these conditions, the antibodies were administered intraperitoneally in three doses of 100 µg applied on days 9, 12 and 15 after the implantation of the tumor cells and the growth of the two concomitant tumor nodes was analyzed. In said experimental conditions, a retarding effect was verified in tumor growth with the treatment with anti-4-1BB and the unilateral intratumoral treatment with the control carrier, resulting in one out of six mice rejecting both tumor nodes. However, the group treated unilaterally with three doses of intratumoral interferon-α and identical intraperitoneal doses of anti-4-1BB monoclonal antibody resulted in a complete bilateral rejection of the tumors in four out of six mice. These data analyzed in survival curves indicate the synergistic effect of both treatments for inducing tumor rejection.

Once it was checked that the combination of treatments improves the antitumor effect of both treatments administered separately, experiments were conducted to clarify the mechanism/mechanisms by means of which the synergistic therapeutic effect takes place. Firstly, experiments were conducted to clarify the cell populations on which the effect of IFNα was necessary. For this purpose, chimeric mice were generated that had bone marrow which came from a donor syngeneic mouse. It was thus possible to obtain a WT mouse with hematopoietic stem cells, which came from bone marrow, that were deficient in type I interferon receptor, and vice versa, a IFNAR^{-/-} knockout mouse with hematopoietic cell lineages that came from a WT mouse.

By following a treatment protocol similar to the one from the previous experiment, it was checked how the deficiency of the type I IFN receptor in the hematopoietic stem cells completely cancelled out the antitumor effect of the antibodies in combination with IFNα (Figure 3a). However, in the opposite situation in which the mice was IFNAR^{-/-} but had received a transplant of normal bone marrow sensitive to type I interferon (Figure 3c), a partial response was observed in the group that received treatment in relation to the results obtained in the control chimeras (Figures 3b and 3d).

These results indicate that the actions exerted by IFNα on the hematopoietic origin cells are critical for the tumor rejection induced by means of the combined treatment strategy between IFNα and anti-4-1BB antibodies.

The comparative analysis of the cell populations in the drainage nodes of the mice subjected to these treatments showed changes dependent thereon relating to a specific immune response (Figure 4a). An increase in the size of the nodes that contained a number of leucocytes 4-5 times higher than that of the tumor-bearing control mice was observed (Figure 4c). It could be verified by means of flow cytometry assays that after the combined treatment, there is a tendency to increase percentage-wise and in absolute numbers the content of T CD8+ lymphocytes that react with the KSPWFTTL (SEQ ID NO:7) tumor antigen shown by the de H2-K^{b} MHC class I molecule. These results were obtained after immunostaining with the tetramer conjugated to PE combined with anti CD8 antibodies (Figure 4a).

The specialized cells responsible for presenting antigens to lymphocytes (such as tumor antigens for example) are dendritic cells. There are dendritic cells classified into different sub-populations in which myeloid or conventional dendritic cells (cDC) and plasmacytoid dendritic cells (pDC) stand out. These cells can be differentiated and listed by means of co-staining with monoclonal antibodies. It is observed from these analyses that both the Intratumor injection of IFNα and the combined IFNα+anti-4-1BB treatment show numeric and percentage increases that can be involved in the therapeutic response (Figure 4b).

To verify the strength of the combined treatment strategy, experimental situations of mice tumors were prepared in which the concomitant presence of tumor lesions transplanted from the MC38 line in the liver and in the subcutaneous tissue of the mice was achieved. Figure 5a shows how the treatment of the subcutaneous lesions with IFNα and the intraperitoneal administration of anti-4-1BB induces the rejection of the hepatic tumors that is not observed in the untreated (control) group. In addition to the absence of hepatic lesions in the exploratory laparotomy, the size of the subcutaneous lesions, which in the experiment shown in Figure 5b were completely rejected in all cases, was tracked, while the tumors of the control group progressed until the mice succumbed due to the effect of the concomitant tumor lesions present in the liver.

## Claims

1. A composition comprising at least one 4-1BB receptor agonist ligand or a functionally equivalent variant thereof and at least one type I interferon or a functionally equivalent variant thereof.

2. Composition according to claim 1, wherein the 4-1BB receptor agonist ligand is the natural ligand of 4-1BB receptor.

3. Composition according to claim 1, wherein the 4-1BB receptor agonist ligand is an anti-4-1BB receptor antibody.

4. Composition according to claim 3, wherein the anti-4-1BB receptor antibody is an IgG-2A type immunoglobulin.

5. Composition according to claims 2, 3 or 4, wherein the anti-4-1BB receptor antibody is a humanized antibody.

6. Composition according to any of claims 1 to 5, wherein the type I interferon is selected from the group comprising interferon-alpha and interferon-beta.

7. Composition according to claim 6, wherein the type I interferon is an interferon-alpha selected from the group of IFN-α2a, IFN-α2b, IFN-α4, IFN-α5, IFN-α8 and combinations thereof.

8. Composition according to any of claims 1 to 7, wherein at least one type I interferon is a pegylated interferon.

9. Composition according to any of claims 1 to 8, further comprising a chemotherapeutic compound.

10. Composition according to any of claims 1 to 9 for its use in medicine.

11. Use of a composition according to any of claims 1 to 9 for preparing a medicament for the treatment or the prevention of cancer.

12. Use according to claim 11, wherein the cancer is a solid tumor.

13. Use according to claim 11, wherein the cancer is a colon carcinoma.

14. A pharmaceutical preparation comprising a composition according to any of claims 1 to 9 and a pharmaceutically acceptable carrier.

15. A kit comprising, in one or several containers,
(i) a pharmaceutically acceptable formulation of at least one 4-1BB receptor agonist ligand, or a functionally equivalent variant thereof,
(ii) a pharmaceutically acceptable formulation of at least one type I interferon, or a functionally equivalent variant thereof, and optionally,
(iii) a pharmaceutically acceptable formulation of at least one chemotherapeutic compound.

16. Kit according to claim 15, wherein the 4-1BB receptor agonist ligand is the natural ligand of 4-1BB receptor.

17. Kit according to claim 16, wherein the 4-1BB receptor agonist ligand is an agonist anti-4-1BB receptor antibody.

18. Kit according to claim 17, wherein the agonist anti-4-1BB receptor antibody is an IgG-2A type immunoglobulin.

19. Kit according to claim 16, 17 or 18, wherein the agonist anti-4-1BB receptor antibody is a humanized antibody.

20. Kit according to any of claims 15 to 19, wherein the type I interferon is selected from the group comprising interferon-alpha and interferon-beta.

21. Kit according to claim 20, wherein the type I interferon is an interferon-alpha selected from the group of IFN-α2a, IFN-α2b, IFN-α4, IFN-α5, IFN-α8 and combinations thereof.

22. Kit according to any of claims 15 to 21, wherein the type I interferon is a pegylated interferon.

23. Kit according to any of claims 15 to 22 for its use in medicine.

24. Kit according to any of claims 15 to 23, further comprising instructions for the simultaneous, sequential or separate administration of the different components.

25. Use of a kit according to any of claims 15 to 24 in the preparation of a medicament for the treatment or the prevention of cancer.

26. Use according to claim 25, wherein the cancer is a solid tumor.

27. Use according to claim 26, wherein the cancer is a colon carcinoma.

28. Use according to any of claims 25 to 27, wherein the separate administration of the different components comprises the systemic administration of the 4-1BB receptor agonist ligand.

29. Use according to any of claims 25 to 29, wherein the separate administration of the different components comprises the intratumoral administration of the type I interferon.

30. Use of a type I interferon, or a functionally equivalent variant thereof, for promoting the anti-tumor effect of a 4-1BB receptor agonist ligand.

31. A polynucleotide comprising
(i) a nucleotide sequence encoding a 4-1BB receptor agonist ligand or a functionally equivalent variant thereof, and
(ii) a nucleotide sequence encoding a type I interferon or a functionally equivalent variant thereof,
wherein both sequences are preceded by expression regulating sequences.

32. Polynucleotide according to claim 31, wherein the 4-1BB receptor agonist ligand is the natural ligand of 4-1BB receptor.

33. Polynucleotide according to claim 31, wherein the 4-1BB receptor agonist ligand is an agonist anti-4-1BB receptor antibody.

34. Polynucleotide according to claim 33, wherein the agonist anti-4-1BB receptor antibody is an IgG-2A type immunoglobulin.

35. Polynucleotide according to claims 32, 33 or 34, wherein the agonist anti-4-1BB receptor antibody is a humanized antibody.

36. Polynucleotide according to any of claims 31 to 35, wherein the type I interferon is selected from the group comprising interferon-alpha and interferon-beta.

37. Polynucleotide according to claim 36, wherein the type I interferon is an interferon-alpha selected from the group of IFN-α2a, IFN-α2b, IFN-α4, IFN-α5, IFN-α8 and combinations thereof.

38. Polynucleotide according to any of claims 31 to 37, wherein the type I interferon is a pegylated interferon.

39. A vector comprising a polynucleotide according to any of claims 31 to 38.

40. A cell comprising a vector according to claim 39.

41. A polynucleotide according to any of claims 31 to 38, a vector according to claim 39 or a cell according to claim 40, for their use in medicine.

42. Use of a polynucleotide according to any of claims 31 to 38, a vector according to claim 39 or a cell according to claim 40 in the preparation of a medicament for the treatment or the prevention of cancer.

43. Use according to claim 42, wherein the cancer is a solid tumor.

44. Use according to claim 42, wherein the cancer is a colon carcinoma.

45. A pharmaceutical preparation comprising a polynucleotide according to any of claims 31 to 38, a vector according to claim 39 or a cell according to claim 40 and a pharmaceutically acceptable carrier.

46. A kit comprising a polynucleotide according to any of claims 31 to 38, a vector according to claim 39 or a cell according to claim 40.

47. Kit according to claim 46 for its use in medicine.

48. Use of a kit according to claim 46 for preparing a medicament in the treatment or prevention of cancer.

49. Use according to claim 48, wherein the cancer is a solid tumor.

50. Use according to claim 48, wherein the cancer is a colon carcinoma.

51. Use of a polynucleotide encoding a type I interferon, or a functionally equivalent variant thereof, for promoting the anti-tumor effect of a 4-1BB receptor agonist ligand.
